# EUROPEAN PATENT APPLICATION

(11) **EP 3 222 205 A1**
(43) Date of publication of application: **27.09.2017**
(21) Application number: 15860596.4
(22) Date of filing: 02.11.2015
(51) Int. Cl.: A61B 5/00

(54) **INFORMATION PROCESSING DEVICE, INFORMATION PROCESSING SYSTEM, INFORMATION PROCESSING METHOD, AND PROGRAM**

(30) Priority: 18.11.2014 US 201462081298 P
(71) Applicant: Sony Corporation, Tokyo 108-0075 (JP)
(72) Inventor: YAJIMA, Masakazu, Tokyo 108-0075 (JP); NIIKURA, Hideo, Tokyo 108-0075 (JP); SUZUKI, Masataka, Tokyo 108-0075 (JP); FUCHIKI, Yayoi, Tokyo 108-0075 (JP)
(74) Representative: Witte, Weller & Partner Patentanwälte mbB
(86) International application number: PCT/JP2015/080959
(87) International publication number: WO 2016/080183

(57) **Abstract**

To detect at least either of the states of an attachment target and a surrounding environment without any time restriction.

Provided is an information processing device including: a power generation signal acquisition unit configured to acquire a power generation signal based on power generation by at least one power generation device; and a determination unit configured to determine at least either of states of an environment surrounding the power generation device and an attachment target to which the power generation device is attached, on the basis of the power generation signal.

## Description

### Technical Field

The present invention relates to an information processing device, an information processing system, an information processing method, and a program.

### Background Art

For example, a monitoring device that uses various sensors to detect information related to a state of an attachment target and a surrounding environment is known.

As such a monitoring device, a monitoring device with a position sensor using radio waves as disclosed in the following Non-Patent Literature 1 can be exemplified, for example.

### Citation List

### Non-Patent Literature

Non-Patent Literature 1: htttp;//www.authjapan.com/#!about/clse

### Disclosure of Invention

### Technical Problem

However, such a monitoring device that uses various sensors consumes electric power for driving the various sensors, converting detected information into signals, and the like. Therefore, it is difficult to continuously drive the monitoring device for a long period of time at a location or in a situation where it is difficult to supply electric power to the monitoring device.

Thus, the present disclosure will propose a novel and improved information processing device, an information processing system, an information processing method, and a program capable of detecting at least either of the states of the attachment target and the surrounding environment without any time restriction.

### Solution to Problem

According to the present disclosure, there is provided an information processing device including: a power generation signal acquisition unit configured to acquire a power generation signal based on power generation by at least one power generation device; and a determination unit configured to determine at least either of states of an environment surrounding the power generation device and an attachment target to which the power generation device is attached, on the basis of the power generation signal.

According to the present disclosure, there is provided an information processing system including: a monitoring device that includes at least one power generation device; and an information processing device that includes a power generation signal acquisition unit configured to acquire a power generation signal based on power generation by the power generation device, and a determination unit configured to determine at least either of states of an environment surrounding the monitoring device and an attachment target to which the monitoring device is attached, on the basis of the power generation signal.

According to the present disclosure, there is provided an information processing method including: acquiring a power generation signal based on power generation by at least one power generation device; and determining, by a computation processing device, at least either of states of an environment surrounding the power generation device and an attachment target to which the power generation device is attached, on the basis of the power generation signal.

According to the present disclosure, there is provided a program that causes a computer to function as: a power generation signal acquisition unit configured to acquire a power generation signal based on power generation by at least one power generation device; and a determination unit configured to determine at least either of states of an environment surrounding the power generation device and an attachment target to which the power generation device is attached, on the basis of the power generation signal.

According to the present disclosure, it is possible to determine at least either of states of an environment surrounding a power generation device and an attachment target to which the power generation device is attached on the basis of power generation by the power generation device without using various sensors that consume electric power.

### Advantageous Effects of Invention

According to the present disclosure, it is possible to detect information related to at least either of the states of the attachment target and the surrounding environment without any time restriction.

Note that the effects described above are not necessarily limitative. With or in the place of the above effects, there may be achieved any one of the effects described in this specification or other effects that may be grasped from this specification.

### Brief Description of Drawings

[FIG. 1] FIG. 1 is an explanatory diagram illustrating an exemplary form of a monitoring device.
[FIG. 2] FIG. 2 is an explanatory diagram illustrating an exemplary power generation method of a power generation device provided in the monitoring device.
[FIG 3] FIG. 3 is an explanatory diagram illustrating an exemplary information processing system that includes the monitoring device.
[FIG. 4] FIG. 4 is an explanatory diagram illustrating an exemplary information processing system that includes the monitoring device.
[FIG. 5] FIG. 5 is a block diagram illustrating an exemplary configuration of the monitoring device.
[FIG. 6] FIG 6 is a block diagram illustrating another configuration example of the monitoring device.
[FIG. 7] FIG. 7 is a block diagram illustrating another configuration example of the monitoring device.
[FIG. 8] FIG. 8 is a block diagram for describing a functional configuration of the information processing system that includes the monitoring device.
[FIG. 9] FIG. 9 is an explanatory diagram illustrating an exemplary determination table for determining at least either of states of an attachment target and a surrounding environment.
[FIG. 10] FIG 10 is a flowchart for describing an operation example of an information processing system according to an embodiment of the present disclosure.
[FIG. 11] FIG. 11 is an explanatory diagram for describing a specific system configuration of the information processing system according to the embodiment of the present disclosure.
[FIG 12] FIG. 12 is an explanatory diagram illustrating a specific configuration of the information processing system when an attachment target is a farm animal.
[FIG. 13] FIG. 13 is an explanatory diagram illustrating an attachment example of a monitoring device when an attachment target is a farm animal.
[FIG 14] FIG. 14 is a flowchart for describing an operation of determining a state of the attachment target by using a power generation signal of the monitoring device.
[FIG. 15] FIG. 15 is a flowchart for describing an operation of determining a state of the attachment target by using a power generation signal of the monitoring device.
[FIG. 16] FIG. 16 is a flowchart for describing an operation of determining a state of the attachment target by using a power generation signal of the monitoring device.
[FIG. 17] FIG. 17 is a flowchart for describing an operation of determining a state of the attachment target by using a power generation signal of the monitoring device.
[FIG. 18] FIG. 18 is a flowchart for describing an operation of determining a state of the attachment target by using a power generation signal of the monitoring device.
[FIG. 19] FIG. 19 is a sequence diagram for describing an operation of determining variation in an environment surrounding the monitoring device on the basis of the power generation signal from the monitoring device.
[FIG. 20] FIG. 20 is a sequence diagram for describing an operation of determining variation in an environment surrounding the monitoring device on the basis of the power generation signal from the monitoring device.
[FIG. 21] FIG. 21 is a sequence diagram for describing an operation of determining variation in an environment surrounding the monitoring device on the basis of the power generation signal from the monitoring device.
[FIG 22] FIG. 22 is a flowchart for describing an operation of determining a state of the attachment target or the surroundings the attachment target by using the power generation signal from the monitoring device and information related to variation in an environment surrounding a receiver.
[FIG. 23] FIG. 23 is a flowchart for describing an operation of determining a state of the attachment target or the surroundings the attachment target by using the power generation signal from the monitoring device and information related to variation in an environment surrounding a receiver.
[FIG. 24] FIG. 24 is a flowchart for describing an operation of determining a state of the attachment target or the surroundings the attachment target by using the power generation signal from the monitoring device and information related to variation in an environment surrounding a receiver.
[FIG. 25] FIG. 25 is a flowchart for describing an operation of determining a state of the attachment target or the surroundings the attachment target by using the power generation signal from the monitoring device and information related to variation in an environment surrounding a receiver.
[FIG. 26] FIG. 26 is a block diagram illustrating a hardware configuration example of an information processing device according to an embodiment of the present disclosure.

### Mode(s) for Carrying Out the Invention

Hereinafter, preferred embodiments of the present disclosure will be described in detail with reference to the appended drawings. In this specification and the appended drawings, structural elements that have substantially the same function and structure are denoted with the same reference numerals, and repeated explanation of these structural elements is omitted.

Now, description will be given in the following order.
1. Detailed description of background
2. Basic configuration example of monitoring device
2-1. Overall outline of system
2-2. Configuration example of monitoring device
2-2-1. Example in which individual computation devices are used
2-2-2. Example in which common computation device is used
2-2-3. Example in which common electrical storage device is used
2-3. Functional configuration example of system
2-4. Operation example of system
2-5. Use example of system
2-5-1. First use example
2-5-2. Second use example
2-6. Specific example of information processing system
3. Hardware configuration example of information processing device
4. Conclusion

### «1. Detailed description of background»

For example, a monitoring device that includes various sensors for detecting a body temperature, a pulse rate (that is, a heart rate), an electrocardiogram, an electromyogram, sweating, an amount of activity, and the like and detects such information is known as a monitoring device for detecting information related to an attachment target that is a living body. Also, a monitoring device that includes various sensors for detecting an internal temperature, a surface temperature, a vibration frequency, a vibration acceleration, and the like and detects such information is known as a monitoring device for detecting information related to an attachment target other than a living body.

Furthermore, a monitoring device that includes various sensors for detecting an air temperature, moisture, illuminance, or a moving direction, a moving velocity, a position, or the like of an attachment target and detects such information is known as a monitoring device for detecting information related to an environment surrounding the attachment target,

However, all the various sensors provided in the monitoring device consume electric power for driving the sensors themselves and performing signal processing such as analog-digital (AD) conversion of the detected information. Therefore, it is difficult to drive these various sensors and to cause the monitoring device to continuously detect states of the attachment target and the surrounding environment at a location or in a situation where it is difficult to supply electric power to the various sensors, due to time restriction.

If the capacity of an electrical storage device, such as a battery, provided in the monitoring device is increased in order to drive the various sensors provided in the monitoring device for a long period of time, it is necessary to further increase the size of the monitoring device. That is, the driving time of the monitoring device depends on the electric power consumed by the various sensors provided in the monitoring device and the size of the monitoring device.

In contrast, the size of the monitoring device is also restricted by the size of the attachment target to which the monitoring device is attached and a situation in which the monitoring device is used. In order for the monitoring device to be attached continuously without causing an uncomfortable feeling in the attachment target, it is necessary that the monitoring device have a small size.

Therefore, a monitoring device that is constantly attached to an attachment target tends to be driven for a shorter period of time. Also, there is a tendency that the driving time becomes shorter as the number of various sensors provided in the monitoring device increases since the power consumption increases. Furthermore, there is a tendency that the frequency of opportunity loss in which the monitoring device cannot be used due to unintentional depletion of the electric power (that is, dead battery) increases as the driving time of the monitoring device becomes shorter.

The aforementioned background makes it difficult to cause the monitoring device to provide information about the state of the attachment target to other persons when the attachment target of the monitoring device has encountered an unexpected disaster, an accident, an incident, or distress, for example. For example, a monitoring device that provides information about a position of the attachment target to other persons at the time of a disaster or the like using a position sensor that uses the global navigation satellite system (GNSS) or the like has limited continuous driving time since the position sensor consumes electric power. Also, it is difficult to provide the information of the state of the attachment target, such as whether the attachment target who has encountered a disaster or the like is in a dangerous situation, to other persons by such a monitoring device.

Furthermore, such a monitoring device requires the attachment target or a person who is in charge of charging the monitoring device with electric power to pay attention to the amount of electric power stored in the monitoring device in order not to invite opportunity loss in which the monitoring device cannot be used due to depletion of electric power when the attachment target has encountered an unexpected disaster, an accident, an incident, or distress. Therefore, such a monitoring device forces a mental burden on the attachment target or the person who is in charge of charging the monitoring device with electric power during carrying or use of the monitoring device.

An information processing device according to an embodiment of the present disclosure uses a power generation device, the amount of power generation of which varies depending on a state of the attachment target or a surrounding environment, rather than various sensors to determine at least either of states of the attachment target or the surrounding environment. Specifically, the information processing device according to the embodiment determines at least either of the states of the attachment target or the surrounding environment on the basis of electric power generated by the power generation device provided in the monitoring device, an output voltage, or an amount of generated power, or amounts of variation in these values.

In this manner, the information processing device according to the embodiment can monitor the state of the attachment target, and the surrounding environment, and the like without causing concern about opportunity loss for use of the monitoring device due to depletion of the electric power and a burden of charging the monitoring device in the attachment target and the like and without any time restriction.

Specifically, the information processing device according to the embodiment can estimate a position of the attachment target by determining the state of the environment surrounding the attachment target. In this manner, the information processing device according to the embodiment can search for an attachment target, such as a sufferer of a disaster, an accident, an incident, or distress or a lost article, for a long period of time, which is difficult to realize with a monitoring device that uses a battery or the like as a power source to drive various sensors. Also, the information processing device according to the embodiment can not only estimate the position of the attachment target but also ascertain the state of the attachment target.

### «2. Basic configuration example of monitoring device»

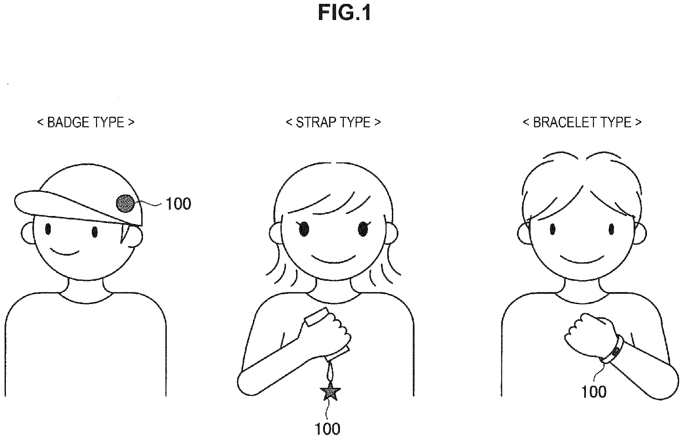

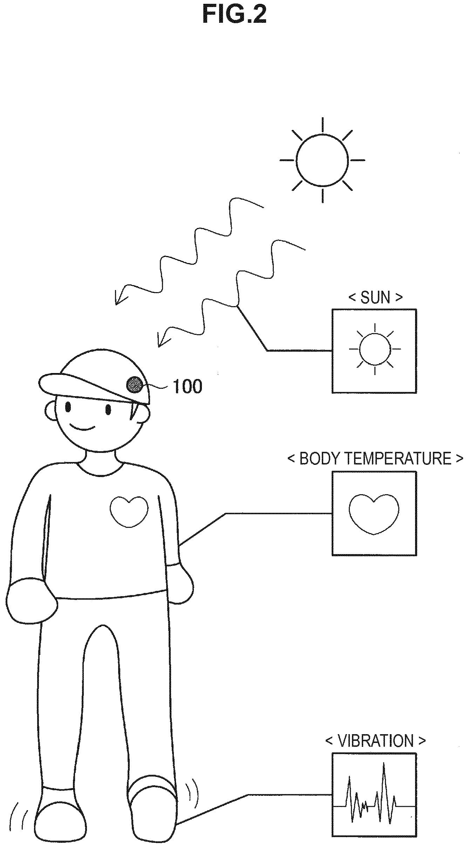

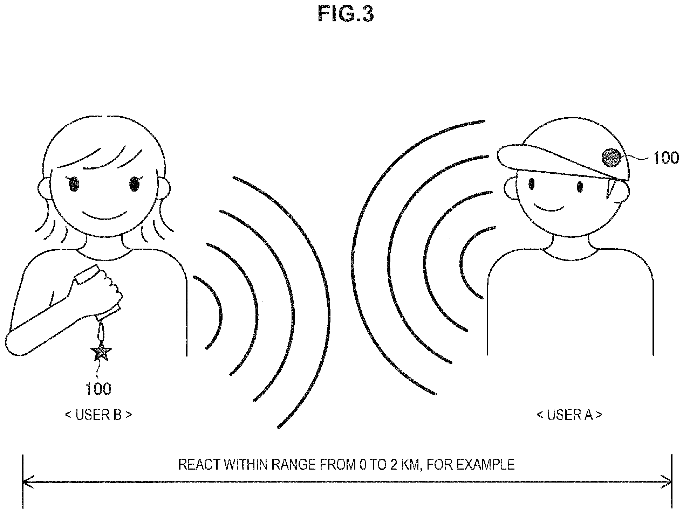

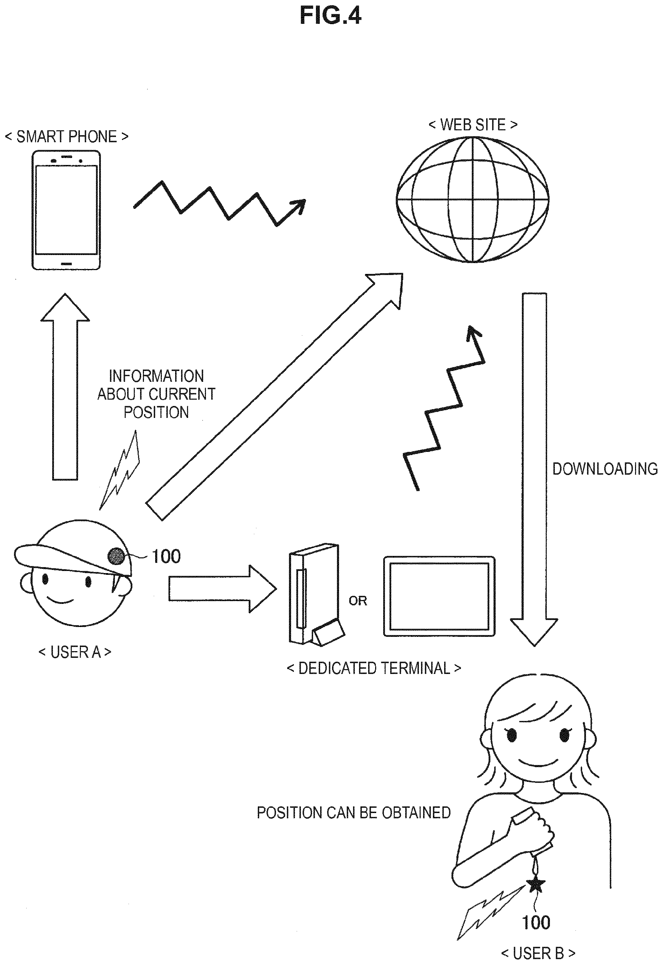

Next, a basic configuration common to information processing systems that include monitoring devices according to the respective embodiments of the present disclosure will be described with reference to FIGS. 1 to 4. FIG. 1 is an explanatory diagram illustrating an exemplary form of a monitoring device 100, and FIG. 2 is an explanatory diagram illustrating an exemplary power generation method of a power generation device provided in the monitoring device 100. FIGS. 3 and 4 are explanatory diagrams illustrating an exemplary information processing system that includes the monitoring device 100. Hereinafter, a configuration example of the information processing system that includes the monitoring device will be described first, and then a configuration example of the monitoring device will be described.

### <2-1. Overall outline of system>

The monitoring device 100 according to the embodiment includes at least one power generation device. The monitoring device 100 is a device that can be attached to or carried by a user or the like and can employ various forms. As illustrated in FIG. 1, for example, the monitoring device 100 may be a badge-type monitoring device 100 that is attached to a cap or the like, a strap-type monitoring device 100 that is attached to a mobile terminal device or the like, or a bracelet-type monitoring device 100 that is attached to a wrist or the like of the user.

As illustrated in FIG 2, for example, the power generation device provided in the monitoring device 100 may be a device that generates power by using solar light, a device that generates power by using a body temperature of the user, or a device that generates power by using vibration that accompanies actions of the user.

The monitoring device 100 may include a communication device, for example, along with the power generation device, thereby enabling communication with another monitoring device 100. As illustrated in FIG. 3, for example, the monitoring device 100 that is attached to a user A and the monitoring device 100 that is attached to a user B may be used as beacon devices. If the monitoring devices 100 are used as beacon devices, two monitoring devices 100 can transmit and receive information to and from each other if the monitoring devices 100 are within a range of a distance of 0 km to 2 km, for example.

If the monitoring devices 100 include communication devices capable of communicating with a communication network such as the Internet, or if the monitoring devices 100 include devices for communication with smart phones or dedicated terminal devices capable of communicating with a communication network such as the Internet, two monitoring devices 100 can transmit and receive information to and from each other via the communication network as illustrated in FIG 4. For example, the user B can acquire information related to the position of the user A by causing the monitoring device 100 carried by the user B to receive the information about the current position of the user A, which has been transmitted from the monitoring device 100 attached to the user A.

Information detected by the monitoring devices 100 may be collected in the information processing device, a database, or the like via the communication network such as the Internet. The dedicated terminal devices may be tablet terminals, personal digital assistances (PDAs), mobile phones including smart phones, devices with a screen such as personal computers, gateway devices with no display screens, and antenna devices installed at predetermined points.

In addition, the monitoring device 100 according to the embodiment may be used in various aspects.

### <2-2. Configuration example of monitoring device>

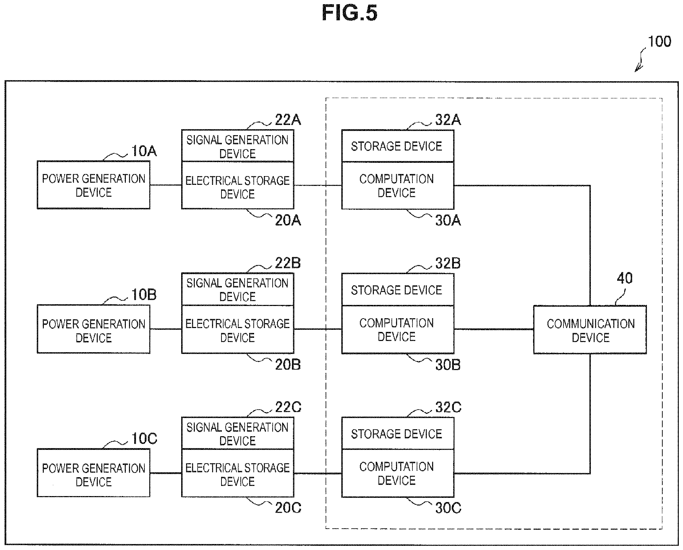

Next, a configuration example of the monitoring device 100 according to the embodiment will be described with reference to FIGS. 5 to 7. The monitoring device 100 according to the embodiment includes at least one power generation device.

### (2-2-1. Example in which individual computation devices are used)

FIG. 5 is a block diagram illustrating an exemplary configuration of the monitoring device 100. As illustrated in FIG. 5, the monitoring device 100 includes three power generation devices 10A, 10B, and 10C, three electrical storage devices 20A, 20B, and 20C, three signal generation devices 22A, 22B, and 22C, three computation devices 30A, 30B, and 30C, three storage devices 32A, 32B, and 32C, and one computation device 40, for example.

### (Power generation devices)

As the power generation devices 10A, 10B, and 10C, any one or a plurality of power generation devices are selected and used from among vibration/kinetic power generation devices, solar power generation devices, thermoelectric conversion power generation devices, enzymatic power generation devices, radio wave power generation devices, near electromagnetic field power generation devices, and electric power transmission devices based on magnetic resonance, electromagnetic induction or electric field coupling, for example. Also, power generation devices other than the types in the above examples may be used as the power generation devices 10A, 10B, and 10C. Although all the power generation devices 10A, 10B, and 10C may be the same type of power generation device, different types of power generation device can acquire various kinds of information related to states of the attachment target and the surrounding environment.

The vibration/kinematic power generation device is configured by using an electrostatic-type, electromagnetic-type, inverse magnetostrictive-type, or piezoelectric-type power generation element, for example, and generates power by using vibration energy or vibration energy. The solar power generation device is configured by using a solar battery, for example, and generates power by using solar light. The thermoelectric conversion power generation device is configured by using a power generation element that uses a Seebeck effect or a Thomson effect, a thermionic power generation element, or a thermomagnetic power generation device, for example, and generates power by using at least one of a body temperature and an ambient temperature. The enzymatic power generation device generates power by decomposing carbohydrates (such as glucose) contained in organic substances with enzymes, for example.

The radio wave power generation device generates power by using radio waves such as Wi-Fi (registered trademark) or a terrestrial digital wave, for example. The near electromagnetic field power generation device generates power by using near field electromagnetic waves, for example. The magnetic resonance power transmission device and the electromagnetic induction power transmission device are devices that use two coils as resonators and are designed such that a current flows through a coil on a power receiving side due to a current flowing through a coil on a power supply side. The electric field coupling power transmission device is a device designed such that a current flows through one of two facing electrode panels due to a high-frequency current flowing through the other electrode panel.

### (Electrical storage devices)

As the electrical storage devices 20A, 20B, and 20C, any one or a plurality of electrical storage devices are selected and used from among capacitors, condensers, secondary batteries, and electrical storage elements with characteristics intermediate therebetween, for example. The electrical storage devices 20A (20B or 20C) are connected to the power generation devices 10A (10B or 10C), respectively and can store electric power generated by the power generation devices 10A (10B or 10C). Also, electrical storage devices other than the types in the above examples may be used as the electrical storage devices 20A, 20B, and 20C. All the electrical storage devices 20A, 20B, and 20C may be the same type of electrical storage devices or may be different types of electrical storage devices.

If the electric power stored in the electrical storage devices 20A, 20B, and 20C is used only for generating a signal that includes information related to at least either of the states of the attachment target and the surrounding environment, the electric power storage capacity of the electrical storage devices 20A, 20B, and 20C may be relatively small. Also, the electric power stored in the electrical storage devices 20A, 20B, and 20C may be used for overall control of the monitoring device 100.

### (Signal generation devices)

The signal generation devices 22A, 22B, and 22C experience state transition on the basis of the electric power generated by the power generation devices 10A, 10B, and 10C, respectively and store the fact of the state transition. As the signal generation devices 22A, 22B, and 22C, switching elements as minimum configurations can be exemplified. The switching element can cause state transition between two states, namely ON and OFF states by supply of the electric power from the power generation devices 10A, 10B, and 10C, for example.

The switching elements may not be able to continuously store the transition state without an energy supply state being maintained or may be able to continuously store the transition state without the energy supply state being maintained. Here, if the switching elements can continuously store the transition state without the energy supply state being maintained, the switching elements themselves correspond to the signal generation devices 22A, 22B, and 22C.

In contrast, if the switching elements cannot continuously store the transition state without the energy supply state being maintained, other elements that can continuously store the transition state without the energy supply state being maintained may be connected to the switching elements. Specifically, if the state transits due to the state transition of the switching elements and an element capable of maintaining the state transition without energy supply is made to store the state transition of the switching elements, the state transition between the two states, namely the ON and OFF states of the switching elements can be stored. In such a case, a series of elements including the switching elements and an element connected to the switching elements correspond to the signal generation devices 22A, 22B, and 22C.

The signal generation devices 22A, 22B, and 22C may be more complicated on the basis of the aforementioned switching operation. For example, the signal generation devices 22A, 22B, and 22C may generate command signals for state transition based on the electric power generated by the power generation devices 10A, 10B, and 10C and execute a group of commands in a unit or units of operations on the assumption that the unit includes a series of operations for achieving a specific purpose. In such a case, processing of storing data and the like is performed along with the processing of executing the aforementioned group of commands, and matters stored in the storage processing may be utilized as storage that represents the state transition. The signal generation devices 22A, 22B, and 22C may separately include counters or the like that stores operation states.

The storage device that stores the aforementioned matters may use the storage devices 32A, 32B, and 32C, and if the communication device 40 or the like includes a storage device, the storage device provided in the communication device 40 may be used. If the storage device provided in the communication device 40 is used, the storage device can store the respective signals based on the electric power generated by the power generation devices 10A, 10B, and 10C by sharing a storage region by time-division or interruption processing.

Specifically, the signal generation device 22A (22B or 22C) generates a signal that includes identification information unique to the power generation device 10A (10B or 10C) and information related to an operation state of a switch circuit such as a reset Integrated Circuit (IC). The generated signal may be stored in the storage device 32A (32B or 32C) or the storage device provided in the communication device 40. The signal generation device 22A (22B or 22C) may generate a signal that further includes information related to properties of the power generation device 10A (10B or 10C) such as an impedance and output properties of the power generation device 10A (10B or 10C).

Furthermore, the signal generation device 22A (22B or 22C) may utilize, as a signal generated, a signal similar to an identification signal necessary for authentication with a communication target of the communication deice 40. That is, if electric power necessary for driving the communication device 40 is stored in the electrical storage device 20A (20B or 20C), the electric power is supplied to the communication device 40 by the reset IC or the like turning on an electric power supply line from the electric storage device 20A to the communication device 40. At this time, the signal generation device 22A (22B or 22C) may generate, as a signal, information necessary for authentication and connection operations with respect to the communication target of the communication device 40.

In such a case, information transmitted from the communication device 40 basically includes identification information for identifying a system of the power generation device 10A (10B or 10C) and whether the signal has been generated by the power generation device 10A (10B or 10C) or the number of times the signal has been generated. Information related to time may be added to the information transmitted from the communication device 40 as needed.

The information transmitted from the communication device 40 is subjected to information processing into the received information related to at least the state of the attachment target to which the monitoring device 100 is attached and the environment surrounding the monitoring device 100 by the computation device provided in the information processing device. However, the information transmitted from the communication device 40 may be information after information processing into the information related to at least either of the state of the attachment target to which the monitoring device 100 is attached and the environment surrounding the monitoring device 100 by the computation devices 30A, 30B, and 30C provided in the monitoring device 100.

### (Computation devices)

The computation devices 30A, 30B, and 30B may be comparators, microcomputers, or the like with simple configurations, for example, though it depends on a purpose of use of the monitoring device 100, and are not particularly limited. All the types of the computation devices 30A, 30B, and 30C may be the same or different from each other.

### (Storage devices)

The storage devices 32A, 32B, and 32C may be volatile storage devices or non-volatile storage devices. For example, the storage devices 32A, 32B, and 32C may be storage elements such as random access memories (RAMs) capable of temporarily storing information. If the storage devices 32A, 32B, and 32C are non-volatile storage devices, the monitoring device 100 can save information in the storage devices 32A, 32B, and 32C before the electric power stored in the electrical storage devices 20A, 20B, and 20C runs out and cause the storage devices 32A, 32B, and 32C to store the information and the state before the electric power runs out. In such a case, other monitoring devices 100 and reading devices can read the information and the state of the monitoring device 100 before the electric power runs out by utilizing a communication technology such as NFC or RFID capable of performing communication even if no electric power is stored in the electrical storage devices 20A, 20B, and 20C. As illustrated in FIG. 5, the storage device 32A (32B or 32C) may be mounted on the computation device 30A (30B or 30C), and the storage device 32A (32B or 32C) and the computation device 30A (30B or 30C) may form a single unit block. The storage devices 32A, 32B, and 32C may form independent unit blocks.

If the computation devices 30A, 30B, and 30C are microcomputers, the storage devices 32A, 32B, and 32C may include storage elements, such as ROMs (Read Only Memories), which stores programs to be executed by the microcomputers. All the types of the storage devices 32A, 32B, and 32C may be the same or different from each other.

### (Communication device)

The communication device 40 is a device that outputs information stored in the monitoring device 100 to the outside and may be a wireless or wired physical signal line. The communication device 40 may be a wireless-type device that uses infrared rays, radio waves, or an electric field, for example. Specifically, the communication device 40 may be a device that can transmit and receive wavelength signals in a band from several hundreds of MHz to several GHz, representative examples of which include Wi-Fi (registered trademark), Zigbee (registered trademark), Bluetooth (registered trademark), Bluetooth Low Energy (registered trademark), ANT (registered trademark), ANT+ (registered trademark), and EnOcean Alliance (registered trademark). Further, the communication device 40 may be a device that can perform near field communication, representative examples of which include NFC. The communication device 40 may include a storage device which is not illustrated in the drawing.

As illustrated by the dashed line in FIG. 5, the aforementioned computation devices 30A, 30B, and 30C may be realized by using computation devices provided in the communication device 40. If the monitoring device 100 is connected to another monitoring device 100, the Internet, or the like via other devices installed in the surroundings thereof, the communication device 40 may be a wired-type device.

### (Other devices)

The monitoring device 100 may also include various devices other than the respective devices illustrated in FIG 5 as needed. For example, the monitoring device 100 may further include a rectifier circuit that rectifies outputs from the power generation devices 10A, 10B, and 10C, regulators that boost or lower output voltages of the power generation devices 10A, 10B, and 10C, charge circuits that control charging and discharge states of the electrical storage devices 20A, 20B, and 20C or perform the aforementioned monitoring, computation devices and storage devices for switching electric power supply to the signal generation devices 22A, 22B, and 22C for signal generation in a more complicated manner, actuators, and display devices such as light emitting diode (LED) lamps and memory-type displays.

As electric power necessary for the signal generation by the signal generation devices 22A, 22B, and 22C, analysis and processing of the generated signals, communication by the communication device 40, driving of an actuator, and display by a display device, the electric power generated by the power generation devices 10A, 10B, and 10C may be utilized. Specifically, predetermined electric power from the electric power generated by the power generation device 10A, 10B, and 10C may be commonly stored in an electrical storage device as a separate system from the electrical storage devices 20A, 20B, and 20C connected to the signal generation devices 22A, 22B, and 22C and may be utilized. Also, a part of the electric power stored in the electrical storage devices 20A, 20B, and 20C connected to the signal generation devices 22A, 22B, and 22C may be utilized. Furthermore, electric power stored in a primary battery or an electrical storage device that has been charged from an external power source may be utilized.

Although the three power generation devices 10A, 10B, and 10C, the three electrical storage devices 20A, 20B, and 20C, the three signal generation devices 22A, 22B, and 22C, the three computation devices 30A, 30B, and 30C, and the three storage devices 32A, 32B, and 32C are provided in the configuration example of the monitoring device 100 illustrated in FIG. 5, the numbers thereof are not limited to three, may be less than or equal to two, or may be more than or equal to four. In the configuration example illustrated in FIG. 5, a plurality of devices appropriately selected from among the power generation devices 10A, 10B, and 10C, the electrical storage devices 20A, 20B, and 20C, the signal generation devices 22A, 22B, and 22C, the computation devices 30A, 30B, and 30C, the storage devices 32A, 32B, and 32C, and the communication device 40 may configure a unit block. For example, the power generation device 10A, the electrical storage device 20A, the signal generation device 22A, the computation device 30A, the storage device 32A, and the communication device 40 may be configured as a single unit block.

If a minimum amount of electric power with which the signal generation devices 22A, 22B, and 22C are driven is stored in the electrical storage devices 20A, 20B, and 20C, signals unique to the systems of the power generation devices 10A, 10B, and 10C are generated in the monitoring device 100 according to the embodiment. The monitoring device 100 according to the embodiment utilizes a combination of the signals generated at this time as sensing information. The aforementioned sensing information may include information indicating that there is no signal (that is, the power generation devices 10A, 10B, and 10C have not generated power).

Specifically, the monitoring device 100 generates a signal (that is, a primary signal) indicating that the power generation devices have generated electrical power and the signal generation devices have been driven by the electric power generated. The information processing device that has received the primary signal can generate information (that is, a secondary signal) related to at least either of the state of the attachment target to which the monitoring device 100 is attached and the environment surrounding the monitoring device 100 on the basis of the primary signal. The information processing device that has received the secondary signal may further generate information (that is, a tertiary signal) related to response to the attachment target from the information related to at least either of the state of the attachment target to which the monitoring device 100 is attached and the environment surrounding the monitoring device 100.

The tertiary signal may be a control command for an external device that is connected to the monitoring device 100, which has generated the tertiary signal, or the information processing device in a wired or wireless manner or an application installed on the external device, or may be a signal of a higher order than a control command, for example. The monitoring device 100 or the information processing device may generate the primary signal or the secondary signal as a control command for an external device connected to the information processing device in a wired or wireless manner or an application installed on the external device.

### (2-2-2. Example in which common computation device is used)

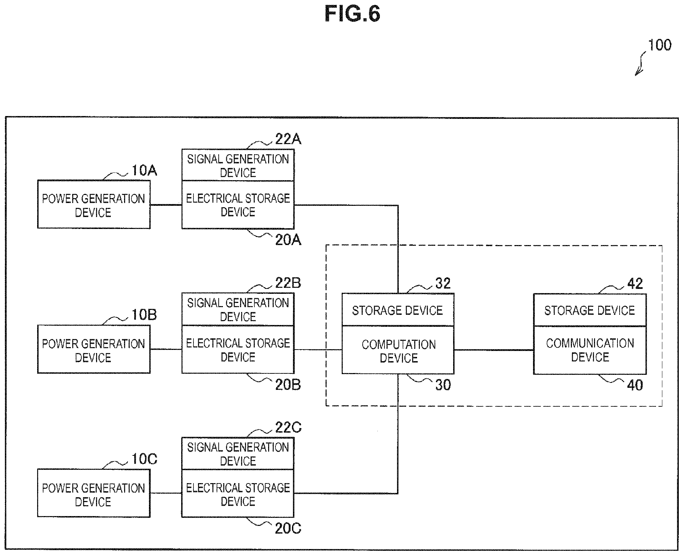

Next, another example of a configuration of the monitoring device 100 will be described with reference to FIG. 6. FIG. 6 is a block diagram illustrating another configuration example of the monitoring device 100.

As illustrated in FIG. 6, the monitoring device 100 includes the three power generation devices 10A, 10B, and 10C, the three electrical storage devices 20A, 20B, and 20C, the three signal generation devices 22A, 22B, and 22C, one computation device 30, a storage device 32 provided in the computation device 30, the one communication device 40, and a storage device 42 provided in the communication device 40, for example. The configuration example of the monitoring device 100 illustrated in FIG. 6 is different from the configuration example of the monitoring device 100 illustrated in FIG. 5 in that only one computation device 30 and one storage device 32 are provided commonly to all the power generation devices 10A, 10B, and 10C and the electrical storage devices 20A, 20B, and 20C are provided.

Here, the basic functions of the respective devices in the configuration example of the monitoring device 100 illustrated in FIG 6 may be the same as those of the respective devices in the configuration example illustrated in FIG. 5. As the storage device 42 provided in the communication device 40, a storage element may be appropriately selected and used from among the storage elements that can be used as the storage device 32 provided in the computation device 30. In the configuration of the monitoring device 100 illustrated in FIG. 6, the computation device 30 may be realized by using the computation device provided in the computation device 40. The storage device 32 can store the respective signals based on the electric power generated by the three power generation devices 10A, 10B, and 10C by sharing a storage region by time-division or interruption processing.

Further, although the three power generation devices 10A, 10B, and 10C, the three electrical storage devices 20A, 20B, and 20C, and the three signal generation devices 22A, 22B, and 22C are provided in the configuration example of the monitoring device 100 illustrated in FIG. 6, the numbers thereof are not limited to three, may be less than or equal to two, or may be more than or equal to four. In the configuration example illustrated in FIG. 6, a plurality of devices appropriately selected from among the power generation devices 10A, 10B, and 10C, the electrical storage devices 20A, 20B, and 20C, the signal generation devices 22A, 22B, and 22C, the computation device 30, the storage device 32, the communication device 40, and the storage device may configure a unit block. For example, the power generation device 10A, the electrical storage device 20A, the signal generation device 22A, the computation device 30, the storage device 32, and the communication device 40 may be configured as a single unit block.

### (2-2-3. Example in which common electrical storage device is used)

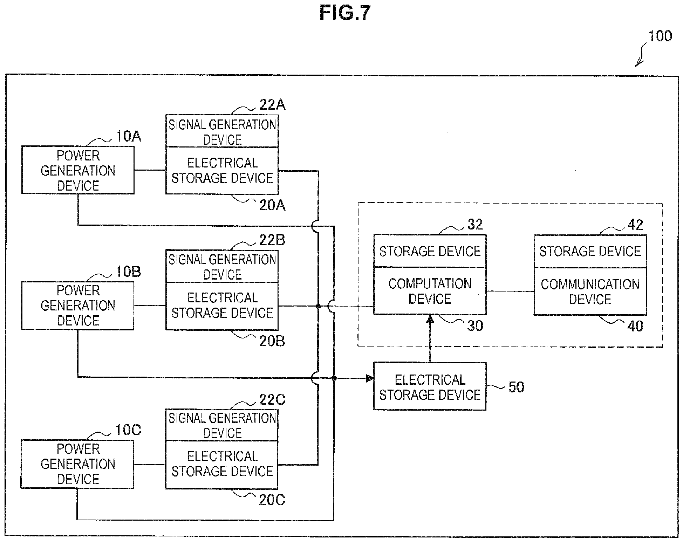

Next, another example of a configuration of the monitoring device 100 will be described with reference to FIG. 7. FIG. 7 is a block diagram illustrating another configuration example of the monitoring device 100.

As illustrated in FIG. 7, the monitoring device 100 includes the three power generation devices 10A, 10B, and 10C, the three electrical storage devices 20A, 20B, and 20C, the three signal generation devices 22A, 22B, and 22C, and the one computation device 30, the storage device 32 provided in the computation device 30, the one communication device 40, the storage device 42 provided in the computation device 40, and an electrical storage device 50 that supplies electric power to the computation device 30 and the communication device 40, for example.

The configuration example of the monitoring device 100 illustrated in FIG. 7 is different from the configuration examples of the monitoring device 100 illustrated in FIG. 6 in that a part of the electric power generated by the power generation devices 10A, 10B, and 10C is stored in the electrical storage device 50 and that the computation device 30 and the communication device 40 are driven by the electric power stored in the electrical storage device 50.

Note that basic functions of the respective devices in the configuration example of the monitoring device 100 illustrated in FIG. 7 may be the same as those of the respective devices in the configuration example illustrated in FIG. 5. Further, the electrical storage device 50 may be appropriately selected and used from among the electrical storage devices that can be used as the electrical storage devices 20A, 20B, and 20C connected to the power generation devices 10A, 10B, and 10C. However, since the electrical storage device 50 stores the electric power for driving the computation device 30 and the communication device 40, a secondary battery or the like with relatively large capacity may be used.

The electrical storage device 50 stores the remaining electric power after removing the electric power necessary for driving the signal generation devices 22A, 22B, and 22C in the electric power generated by the power generation devices 10A, 10B, and 10C. The electrical storage device 50 can more efficiently supply the generated electric power to the computation device 30 and the communication device 40 by accumulating the electric power other than the electric power necessary for the signal generation devices 22A, 22B, and 22C to generate signals.

Although the three power generation devices 10A, 10B, and 10C, the three electrical storage devices 20A, 20B, and 20C, and the three signal generation devices 22A, 22B, and 22C are also provided in the configuration example of the monitoring device 100 illustrated in FIG. 7, the numbers thereof are not limited to three, may be less than or equal to two, or may be greater than or equal to four. In the configuration example illustrated in FIG. 7, a plurality of devices appropriately selected from among the power generation devices 10A, 10B, and 10C, the electrical storage devices 20A, 20B, and 20C, the signal generation devices 22A, 22B, and 22C, the computation device 30, the storage device 32, the communication device 40, and the storage device 42 can also configure a unit block. For example, the power generation device 10A, the electrical storage device 20A, the signal generation device 22A, the computation device 30, the storage device 32, and the communication device 40 may be configured as a single unit block.

The basic configurations of the monitoring device 100 according to the embodiment were described hitherto.

### <2-3. Functional configuration example of system>

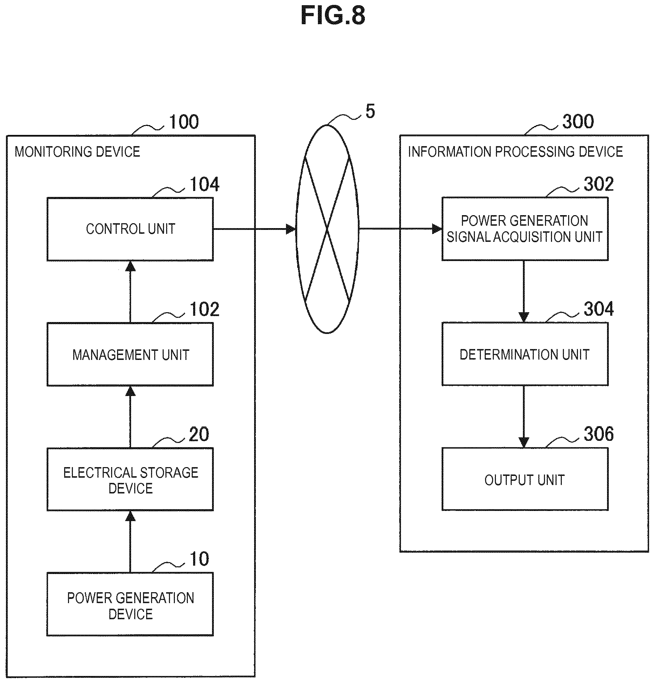

Next, a functional configuration example of an information processing system according to the embodiment will be described with reference to FIG 8. FIG. 8 is a block diagram for describing a functional configuration of the information processing system that includes the aforementioned monitoring device 100.

As illustrated in FIG. 8, the information processing system according to the embodiment includes the monitoring device 100 that is attached to the attachment target and an information processing device 300 that communicates with the monitoring device 100 via a communication network 5.

The monitoring device 100 includes a power generation device 10, an electrical storage device 20, a management unit 102, and a control unit 104, and the information processing device 300 includes a power generation signal acquisition unit 302, a determination unit 304, and an output unit 306.

### (Monitoring device)

The power generation device 10 is a device that generates electric power by using various methods. The electrical storage device 20 is a device that stores the electric power generated by the power generation device 10. The numbers of the power generation devices 10 and the electrical storage devices 20 may be at least one, and upper limits thereof are not particularly limited. Since details of the power generation device 10 and the electrical storage device 20 are as described above, description thereof will be omitted here.

The management unit 102 manages the electric power stored in the electrical storage device 20. Specifically, the management unit 102 checks the electric power stored in the electrical storage device 20 and determines whether or not the electrical storage device 20 has stored electric power that is greater than or equal to a threshold value. The threshold value used by the management unit 102 as reference for the determination herein may correspond to the electric power necessary for performing operations of generating the power generation signal that indicates that the power generation device 10 has generated the electric power and transmitting the power generation signal to the information processing device 300, for example. If the management unit 102 determines that the electrical storage device 20 has stored electric power that is greater than or equal to the threshold value, the signal generation device 22 (22A, 22B, and 22C) generates the power generation signal indicating that the power generation device 10 has generated the electric power, for example. The functions of the management unit 102 may be executed by the computation device 30 (30A, 30B, and 30C).

The control unit 104 controls inputs from the outside to the monitoring device 100 and outputs from the monitoring device 100 to the outside. Specifically, the control unit 104 outputs the power generation signal generated by the signal generation device 22 (22A, 22B, and 22C) to the information processing device 300 via the communication network 5. If there is an input, such as a control command, of a tertiary signal or the like from the information processing device 300 to the monitoring device 100, the control unit 104 receives the input and controls the monitoring device 100 on the basis of the input. The functions of the control unit 104 may be executed by the computation device 30 (30A, 30B, and 30C) and the computation device provided in the communication device 40, for example.

### (Communication network)

The communication network 5 is a network through which communication is performed by the aforementioned communication device 40. The communication network 5 may be a public network such as the Internet, a satellite communication network, a telephone line network, or a mobile communication network (for example, a 3G line network), for example.

### (Information processing device)

The power generation signal acquisition unit 302 acquires the power generation signal transmitted from the monitoring device 100. Specifically, the power generation signal acquisition unit 302 receives the power generation signal that includes information related to the power generation by the power generation device 10 provided in the monitoring device 100 via the communication network 5. The power generation signal acquisition unit 302 is a communication interface configured of a communication device for establishing connection with the communication network 5, for example. The power generation signal acquisition unit 302 may be a communication device that is compatible with a wired or wireless local area network (LAN), may be a cable communication device that performs wired cable communication, or may be an antenna communication device that performs wireless communication using radio waves.

The determination unit 304 determines at least either of the states of the attachment target to which the power generation device 10 is attached and the environment surrounding the power generation device 10 on the basis of the power generation signal received from the monitoring device 100. The attachment target to which the power generation device 10 is attached is substantially the same as the attachment target to which the monitoring device 100 is attached, and the environment surrounding the power generation device 10 is substantially the same as the environment surrounding the monitoring device 100.

Specifically, the determination unit 304 determines the power generation state of the power generation device 10 on the basis of at least information about the amount of electric power generated by the power generation device 10 or an output voltage included in the power generation signal. Also, the determination unit 304 determines at least either of the states of the attachment target to which the power generation device 10 is attached and the environment surrounding the power generation device 10 on the basis of the power generation state of the power generation device 10.

If the amount of electric power generated by the power generation device 10, which is a solar power generation device, is large, for example, the determination unit 304 may determine that the power generation device 10 is located outdoors. If the amount of electric power generated by the power generation device 10, which is a vibration power generation device, is large, the determination unit 304 may determine that the attachment target to which the power generation device 10 is attached is moving or working out. Furthermore, if the amount of electric power generated by the power generation device 10, which is a radio wave power generation device, is small, the determination unit 304 may determine that there are no electronic devices and equipment that emit radio waves around the power generation device 10.

If the power generation device 10 is a radio wave power generation device, the determination unit 304 can determine whether or not the power generation device 10 is located near communication equipment that has emitted a beacon, by causing the communication equipment installed at a predetermined point to emit a beacon. In such a case, the determination unit 304 can acquire information related to the position of the power generation device 10 and can thus determine at least either of the states of the attachment target to which the power generation device 10 is attached and the environment surrounding the power generation device 10 in more detail.

The determination unit 304 may differentiate information about the amount of the electric power generated by the power generation device 10, the output voltage thereof, or the like included in the power generation signal and determine at least either of the states of the attachment target to which the power generation device 10 is attached and the environment surrounding the power generation device 10 on the basis of the amount or the rate of variation in the amount of the electric power generated. Furthermore, the determination unit 304 may determine at least either of the states of the attachment target to which the power generation device 10 is attached and the environment surrounding the power generation device 10 on the basis of a value obtained by integrating the information about the amount of the electric power generated by the power generation device 10, the output voltage thereof, or the like included in the power generation signal. Since the amount of information that can be used by the determination unit 304 for the determination increases in this case, the determination unit 304 can determine at least either of the states of the attachment target to which the power generation device 10 is attached and the environment surrounding the power generation device 10 in more detail.

Furthermore, if the monitoring device 100 is designed to transmit the power generation signal when the electric power generated by the power generation device 10 has exceeded a threshold value, the determination unit 304 may determine a degree of the power generation by the power generation device 10 on the basis of a reception frequency (that is, a generation interval) of the power generation signal. If the reception frequency of the power generation signal is high, the determination unit 304 can determine that the amount of the electric power generated by the power generation device 10 is large.

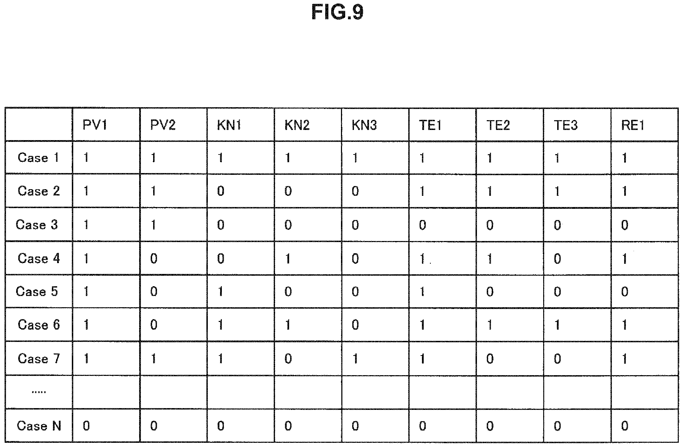

The determination unit 304 may determine at least either of the states of the attachment target to which the power generation device 10 is attached and the environment surrounding the power generation device 10 by combining power generation signals from a plurality of power generation devices 10. For example, the determination unit 304 may prepare a determination table as illustrated in FIG. 9 in advance and determine at least either of the states of the attachment target to which the power generation device 10 is attached and the environment surrounding the power generation device 10 on the basis of a combination of power generation devices 10 that have generated large amounts of electric power. FIG. 9 is an explanatory diagram illustrating an exemplary determination table for determining at least either of the states of the attachment target to which the power generation device 10 is attached and the environment surrounding the power generation device 10.

In the determination table illustrated in FIG. 9, the case where the amount of generated power exceeds the threshold value is represented as "1", and the case where the amount of generated power is less than the threshold value is represented as "0". Here, "PV1" and "PV2" in FIG. 9 represent whether the amount of electric power generated by the power generation device 10, such as a solar power generation device, which generates electric power on the basis of at least either of an absorption wavelength and light intensity has exceeded the threshold value. "KN1", "KN2", and "KN3" represent whether or not the amount of electric power generated by the power generation device 10, such as a vibration/kinetic power generation device, which generates electric power on the basis of at least either of a resonance frequency and vibration intensity has exceeded the threshold value. "TE1", "TE2", and "TE3" represent whether or not the amount of the electric power generated by the power generation device 10, such as a thermoelectric conversion power generation device, which generates electric power on the basis of at least either of a heat capacity and a temperature, has exceeded the threshold value. "RE1" represents whether or not the amount of the electric power generated by the power generation device 1, such as a radio wave power generation device, which generates electric power on the basis of a radio wave frequency and radio wave intensity, has exceeded the threshold value.

The determination table illustrated in FIG. 9 stores, as "Case1" to "CaseN", correspondence between combinations of the respective power generation devices 10 and whether or not the amounts of the electric power generated by the power generation devices 10 have exceeded the threshold value and what the states of the attachment target or the environment surrounding the attachment target were like. If such a determination table is stored in advance, the determination unit 304 can determine at least either of the states of the attachment target to which the power generation device 10 is attached and the environment surrounding the power generation device 10 from the respective amounts of the electric power generated by each power generation devices 10 through simpler information processing on the basis of the determination table.

Furthermore, the determination unit 304 may determine at least either of the states of the attachment target to which the power generation device 10 is attached and the environment surrounding the power generation device 10 on the basis of a temporal variation in the amount of the electric power generated by the power generation device 10 attached to the same attachment target and a difference in the amounts of the electric power generated by the power generation devices 10 attached to different attachment targets.

Specifically, the determination unit 304 may compare the amount of the electric power generated by the power generation device 10 attached to the same attachment target with history data in the past and determine that abnormality has occurred in the attachment target if the amount of the electric power generated by the power generation device 10 shows an abnormal value.

The determination unit 304 may compare the amounts of the electric power generated by the power generation devices 10 attached to different attachment targets and determine that abnormality has occurred in an attachment target if the amount of the electric power generated by the power generation device 10 is abnormally high or low as compared with those of the power generation devices 10 attached to the other attachment targets. Since there is a possibility that there are individual differences in the power generation devices 10 attached to the different attachment targets due to variation in fabrication, the values of the amounts of the electric power generated by the respective power generation devices 10 may be corrected by using a normal distribution, a histogram, and the like for each population.

Furthermore, the determination unit 304 may perform mechanical learning by providing feedback about what kind of abnormality has actually occurred in the attachment target with respect to the abnormality in the amounts of the electric power generated by the power generation devices 10. If a predetermined amount of data samples has been stored, the determination unit 304 can automatically determine what kind of abnormality has occurred in an attachment target with respect to abnormality in the amounts of the electric power generated by the power generation devices 10.

The output unit 306 outputs a signal (for example, a secondary signal or a tertiary signal) generated by the determination unit 304 to an external device that is connected to the information processing device 300 in a wired or wireless manner. The signal output from the output unit 306 may be a control command for the external device to which the signal has been output or an application installed on the external device or may be a higher order signal than the control command. For example, the output unit 306 may output an alert signal indicating that abnormality in the attachment target has been detected.

The output unit 306 may be a connection interface configured of a connection port for establishing connection with the external device, such as a universal serial bus (USB) port, an Ethernet (registered trademark) port, an IEEE80671 standard port, or an optical audio terminal, or may be a communication interface configured of a communication device or the like for establishing connection with the network 5 of a communication device that is compatible with a wired or wireless LAN or the like.

The entirety or a part of the functions of the power generation signal acquisition unit 302, the determination unit 304, and the output unit 306 may be executed by the monitoring device 100. In such a case, the entirety or a part of the functions of the power generation signal acquisition unit 302, the determination unit 304, and the output unit 306 may be executed by the computation device 30 (30A, 30B, and 30C), for example.

The functional configuration example of the information processing system according to the embodiment was described in detail hitherto.

### <2-4 Operation example of system>

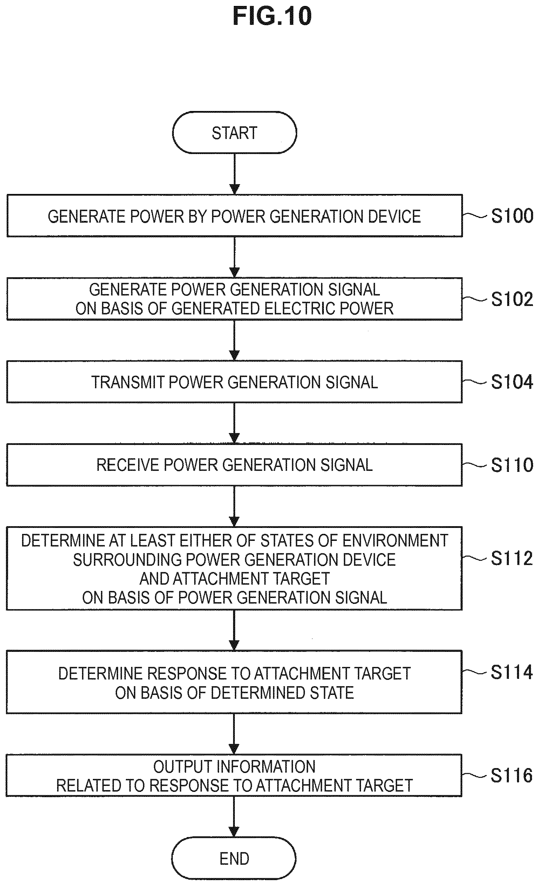

Next, an operation example of the information processing system according to the embodiment will be described with reference to FIG. 10. FIG. 10 is a flowchart for describing an operation example of the information processing system according to the embodiment. The operation example of the information processing system described below is only an example for describing operations of the information processing system according to the embodiment, and operations of the information processing system according to the embodiment are not limited to the following example.

As illustrated in FIG 10, each power generation device 10 in the monitoring device 100 generates electric power first (S100). Then, the management unit 102 and the like generates a power generation signal on the basis of the electric power generated by the power generation device 10 (S102). Subsequently, the control unit 4 transmits the generated power generation signal to the information processing device 300 (S104).

The power generation signal acquisition unit 302 in the information processing device 300 receives the transmitted power generation signal (S110). Then, the determination unit 304 determines at least either of the states of the attachment target to which the power generation device 10 is attached and the environment surrounding the power generation device 10 on the basis of the transmitted power generation signal (S112). Subsequently, the determination unit 304 determines response to the attachment target on the basis of at least either of the states of the attachment target and the surrounding environment (S114). Furthermore, the output unit 306 outputs, to an external device or the like, information related to the response to the attachment target on the basis of the determined response to the attachment target (S114).

The aforementioned operations make it possible for the information processing system according to the embodiment to determine at least either of the states of the environment surrounding the power generation device and the attachment target to which the power generation device is attached on the basis of the power generation by the power generation device without using various sensors that consume electric power.

### <2-5. Use example of system>

Next, a specific use example of the aforementioned information processing system according to the embodiment will be described. The specific use example of the information processing system according to the embodiment described below is only an example for describing the information processing system according to the embodiment, and examples of use of the information processing system according to the embodiment are not limited to the following example.

Specifically, the monitoring device 100 may include, as the power generation devices 10, a Si-based solar power generation device, a dye sensitized solar power generation device, a piezoelectric power generation device, an electromagnetic induction power generation device, a temperature difference power generation device, a thermal responsiveness difference power generation device, and a radio wave power generation device. The electrical storage devices 20 and the signal generation devices 22 such as micro-processing units (MPUs) or the computation devices 30 are independently connected to the respective power generation devices 10. All-solid-state secondary batteries, capacitors, or the like can be used as the electrical storage devices 20, and the electrical storage devices 20 supply the electric power generated by the power generation devices 10 to the signal generation devices 22 or the computation devices 30 respectively connected.

The signal generation devices 22 or the computation device 30, to which the electric power has been supplied, consume the supplied electric power, thereby storing the number of times the electric power has been supplied in either of the storage devices 32 and 42. The signal generation devices 22 and the computation devices 30 may store whether or not the electric power has been supplied as an ON/OFF binary in either of the storage devices 32 and 42.

Furthermore, a common communication device 40 is connected to the respective signal generation devices 22 or the computation devices 30. The electric power generated by the power generation devices 10 is supplied to the communication device 40 via the electrical storage devices 20 after the electric power is supplied to the signal generation devices 22 or the computation devices 30. Information related to the number of times the generated electric power has been supplied to the respective signal generation devices 22 or the computation devices 30 or whether or not the electric power has been supplied is stored in either of the storage devices 32 and 42. The computation device 40 transmits the information stored in the storage devices 32 and 42 to the information processing device 300 and the like when the communication device 40 is driven. The communication device 40 may further transmit identification information unique to the monitoring device 100 to the information processing device 300 and the like when the information related to the number of times the electric power has been supplied or whether or not the electric power has been supplied is transmitted.

The information processing device 300, which has received the transmitted information, performs computation processing on the received information, thereby further generating a secondary signal and a tertiary signal. Furthermore, the information processing device 300 outputs an analysis result in accordance with an external device and an application installed on the external device and a control command accompanying the analysis result as the secondary signal and the tertiary signal.

### (2-5-1. First use example)

For example, the first use example of the information processing system according to the embodiment is directed to a search system for searching for a location of a sufferer or a victim who has encountered a disaster, an incident, an accident, or distress, for example.

For example, the information processing device 300 can acquire a primary signal indicating whether or not electric power sufficient for generating a signal has been generated in each of the power generation devices 10 such as the solar power generation device, the vibration power generation device, the thermoelectric conversion power generation device, the radio wave power generation device, and the enzymatic power generation device provided in the monitoring device 100. The information processing device 300 can generate a secondary signal related to a state of the attachment target to which the monitoring device 100 is attached or an environment surrounding the monitoring device 100 on the basis of the acquired primary signal.

Specifically, if the solar power generation device has generated sufficient electric power for generating the signal, the signal has been generated, and whether or not the signal has been generated varies between day time and night time, the information processing device 300 can generate a secondary signal indicating that the environment surrounding the attachment target is under the sun rather than out of the sun and there are a small number of or no objects between the attachment target and the sun. If the vibration power generation device has not generated sufficient electric power for generating the signal and the signal has not been generated, the information processing device 300 can generate a secondary signal indicating that the amount of activity of the attachment target has decreased.

If the thermoelectric conversion power generation device has generated sufficient electric power for generating the signal and the signal has been generated, the information processing device 300 can generate a secondary signal indicating that metabolism has continued in the attachment target and life activity has been observed. If the radio wave power generation device has not generated sufficient electric power for generating the signal and the signal has not been generated, the information processing device 300 can generate a secondary signal indicating that there are neither electronic devices that emit radio waves nor infrastructures that emit radio waves in the environment surrounding the attachment target. Furthermore, if the enzymatic power generation device has not generated sufficient electric power for generating the signal and the signal has not been generated, the information processing device 300 can generate a secondary signal indicating that a blood glucose level of the attachment target has decreased or there is no nourishment in the environment surrounding the attachment target.

Furthermore, the information processing device 300 may further generate a tertiary signal by computation processing on the basis of a group of the aforementioned secondary signals. The tertiary signal may be a signal of a priority order indicating that the attachment target needs to be urgently rescued, that the attachment target needs to be rescued though it is not urgent, and that the attachment target does not need to be rescued, for example.

Conditions under which the power generation devices 10 can generate electric power differ depending on materials and shapes of the power generation devices 10. For example, an absorption wavelength with which the solar power generation device can generate electric power, a resonance frequency with which the vibration power generation device can generate electric power, a temperature range and heat capacity with which the thermoelectric power generation device can generate electric power, a substance with which the enzymatic power generation device can generate electric power, and an absorption wavelength with which the radio wave power generation device can generate electric power differ depending on materials and shapes of the power generation devices 10. Therefore, it is possible to detect and express at least either of the state of the attachment target and the environment surrounding the attachment target in a more complicated manner by using power generation devices 10 of a plurality of types even if the power generation devices 10 use the same power generation method, for example, by using an Si-based solar power generation device and a dye sensitized solar power generation device together in the case of solar power generation devices.

### (2-5-2. Second use example)

The second use example of the information processing system according to the embodiment is directed to a lost article search system that is designed such that the attachment target of the monitoring device 100 is a lost article rather than a living body and that is for finding a state of the lost article and a surrounding environment.

For example, the information processing device 300 can acquire a primary signal indicating whether or not the respective power generation devices 10 such as the solar power generation device, the vibration power generation device, the thermoelectric conversion power generation device, and the radio wave power generation device provided in the monitoring device 100 have generated sufficient electric power for generating signals. The information processing device 300 can generate a secondary signal related to the state of the attachment target (that is, the lost article) to which the monitoring device 100 is attached or the environment surrounding the monitoring device 100 on the basis of the acquired primary signal.

Specifically, if the solar power generation device has generated sufficient electric power for generating the signal, the signal has been generated, and whether the signal has been generated varies between day time and night time, the information processing device 300 can generate a secondary signal indicating that the environment surrounding the attachment target is under the sun rather than out of the sun and there are a small number of or no objects between the attachment target and the sun. If the vibration power generation device has not generated sufficient electric power for generating the signal and the signal has not been generated, the information processing device 300 can generate a secondary signal indicating that the attachment target is not in a moving body and is not moving or there are no vibration sources that generate a resonance frequency of the vibration power generation device near the attachment target.

If the thermoelectric conversion power generation device has generated sufficient electric power for generating the signal and the signal has been generated, the information processing device 300 can generate a secondary signal indicating presence thereof in an environment with a temperature difference, for example, in an environment where either of the surfaces of the attachment target is in contact with a substance with different heat capacity or the attachment target is attached to another person. If the radio wave power generation device has not generated sufficient electric power for generating the signal and the signal has not been generated, the information processing device 300 can generate a secondary signal indicating that there are neither electronic devices that emit radio waves nor infrastructures that emit radio waves in the environment surrounding the attachment target.

Furthermore, the information processing device 300 may further generate a tertiary signal by computation processing on the basis of a group of the aforementioned secondary signals. The tertiary signal may include, for example, information about what kind of environment the lost article is in, information for notification of the fact that the lost article has been stolen, and information related to a grade for deciding insurance payment for valuables stored in the lost article.

Conditions under which the power generation devices 10 can generate electric power differ depending on materials and shapes of the power generation devices 10. For example, an absorption wavelength with which the solar power generation device can generate electric power, a resonance frequency with which the vibration power generation device can generate electric power, a temperature range and heat capacity with which the thermoelectric power generation device can generate electric power, and an absorption wavelength with which the radio wave power generation device can generate electric power differ depending on materials and shapes of the power generation devices 10. Therefore, it is possible to detect and express at least either of the state of the attachment target and the environment surrounding the attachment target in a more complicated manner by using power generation devices 10 of a plurality of types even if the power generation devices 10 use the same power generation method, for example, by using an Si-based solar power generation device and a dye sensitized solar power generation device together in the case of solar power generation devices.

### (2-6. Specific example of information processing system)

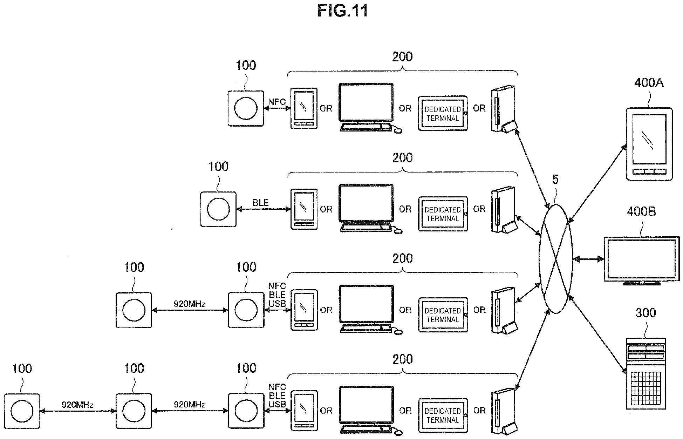

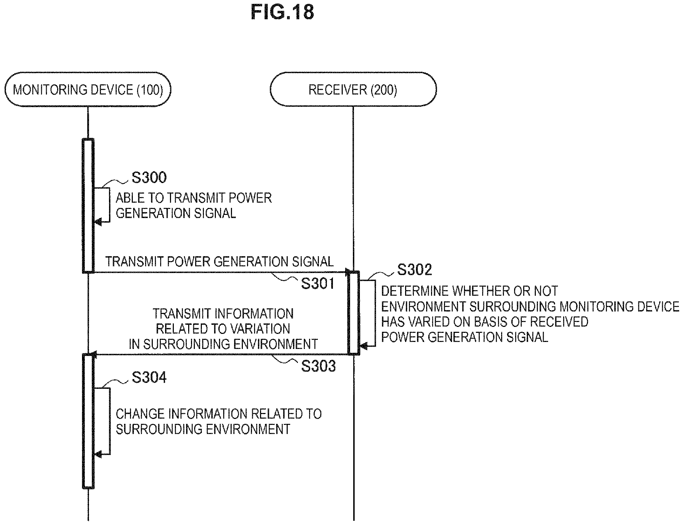

Furthermore, a specific example of the aforementioned information processing system according to the embodiment will be described in detail with reference to FIGS. 11 to 25. FIG. 11 is an explanatory diagram for describing a specific system configuration of the information processing system according to the embodiment.

As illustrated in FIG. 11, the information processing system according to the embodiment includes, for example, small-sized tag-shaped monitoring devices 100, receivers 200 that receive power generation signals from the monitoring devices 100, the information processing device 300 connected to the receivers 200 via the communication network 5, and display devices 400A and 400B for displaying information from the monitoring devices 100 to a user.

Any communication scheme may be employed between the monitoring devices 100 and the receivers 200. As available communication schemes, communication schemes such as near field communication (NFC) for performing proximity-based communication over about several cm by using a frequency of 13.56 MHz, communication schemes such as Wi-Fi, Bluetooth, and Bluetooth Low Energy (BLE) for communication within a range of about 50 m by using a frequency of 2.4 GHz, and communication schemes for communication within a range of about 1 km by using a frequency of 920 MHz can be exemplified.

In the information processing system according to the embodiment, the plurality of monitoring devices 100 may perform communication with each other, and the monitoring devices 100 may be used as relay devices for the communication. In such a case, an optimal communication scheme can be selected in accordance with a role, and for example, the communication scheme using the frequency of 920 Hz for enabling communication between relatively long distances is used for the communication between the monitoring devices 100, and a wired or wireless communication schemes, such as NFC, BLE, and USB, for enabling communication between relatively short distances are used for the communication between the receivers 200 and the monitoring devices 100.

If the receivers 200 are mobile phones, smart phones, tablet terminals, gateway devices, or laptop personal computers and the like and the receivers 200 and the monitoring devices 100 are connected by USBs, for example, the monitoring devices 100 can receive electric power necessary for the communication from the receivers 200. The receivers 200 may not include communication interfaces for communicating with the monitoring devices 100 and may communicate with the monitoring devices 100 via the monitoring devices 100 connected by the USBs.

The receivers 200 are connected to the communication network 5 such as the Internet, a satellite communication network, a telephone line network, and a mobile communication network (a 3G line network or a 4G line network, for example) and transmit the power generation signals, which have been received from the monitoring devices 100, to the information processing device 300.

The information processing device 300 can determine at least either of states of attachment targets to which the monitoring devices 100 are attached and environments surrounding the monitoring devices 100 by performing the aforementioned information processing on the basis of the received power generation signals.

At least either of the states of the attachment targets to which the monitoring device 100 are attached and the environments surrounding the monitoring devices 100, which have been determined by the information processing device 300, can be checked through the display devices 400A and 400B and the like of mobile phones, smart phones, tablet terminals, laptop personal computers, or the television devices, for example.

With such a configuration, the information processing system according to the embodiment can determine at least either of the states of the attachment targets to which the monitoring devices 100 are attached and the environments surrounding the monitoring devices 100 and manage the respective attachment targets. The information processing device 300 can receive the power generation signals from the respective monitoring devices 100 even when the monitoring devices 100 are present in a wide range, by receiving the power generation signals from the monitoring devices 100 via the receivers 200.

The information processing system according to the embodiment can be used for purposes of management of articles to be delivered in logistics, management of animals such as farm animals and pets, monitoring of seniors, dementia patients, children, adults in general, and the like, and entertainments such as attractions, for example.

If the information processing system according to the embodiment is used for managing articles to be delivered in logistics, for example, the monitoring devices 100 are management tags for the articles to be delivered, and the receivers 200 are installed at the respective logistics bases, for example.

If the information processing system according to the embodiment is used for managing farm animals (for example, cattle) and the like, the monitoring devices 100 are search tags attached to the farm animals, and the receivers 200 are installed at a grassland, a shed, a watering place, a milking site, a feeding site, an office, and the like, for example.

If the information processing system according to the embodiment is used for the purpose of monitoring persons, the monitoring devices 100 are search tags or monitoring tags attached to the persons, and the receivers 200 are installed at public facilities such as parks, schools, government offices, police stations, community centers, museums, public day-care centers, and hospitals, private facilities such as factories, department stores, shopping malls, attraction facilities, private day-care centers, and cramming schools, and transportation start points such as stations, bus stops, intersections, transportation signals, ports, airports, mountain huts, and trailheads. The receivers 200 may be installed at home, in the respective regions of the aforementioned respective facilities, and at arbitrary locations such as monitoring points.

Furthermore, if the information processing system according to the embodiment is used for the purpose of entertainments such as attractions, the receivers 200 are installed at entertainment facilities, attraction facilities, the respective venues in these facilities, monitoring points, and event occurring points (that is, locations where events occur by passing through the locations or by reaching the locations). The receivers 200 may be installed in spaces in nature such as parks, public roads, general facilities, hills and fields, rivers, lakes, swamps, and seas rather than the entertainment facilities and the attraction facilities, and the locations where the receivers 200 are installed may be employed as monitoring points or event occurring points. In such a case, any spaces can be places where entertainments such as attractions or events are provided.

The monitoring devices 100 may be registered in association with information related to the attachment targets in the information processing device 300 and the like. If the attachment targets are farm animals, for example, identification information of the monitoring devices 100 may be registered in association with information such as individual identification numbers of the farm animals as the attachment targets, and groups to which the farm animals belong, markings on bodies, and a breeding history. If the attachment targets are persons, the identification information of the monitoring devices 100 may be registered in association with information such as names, ages, addresses, face photos, past illnesses, and medical histories of the persons as the attachment targets.

Identification information of the receivers 200 may be similarly registered in the information processing device 300 and the like. For example, the identification information of the receivers 200 may be registered in association with identification information of communication devices provided in the receivers 200, the positions of the receivers 200, and roles of the receivers 200.

If the attachment targets are persons, the registered information related to the monitoring devices 100 and the receivers 200 may be protected with high information security from the viewpoint of privacy protection and the like. For example, access authority for matching registered information of the monitoring devices 100 and the receivers 200 may be set, and the registered information of the monitoring devices 100 and the receivers 200 may be encrypted. The registered information of the monitoring devices 100 and the receivers 200 may be unitarily managed.

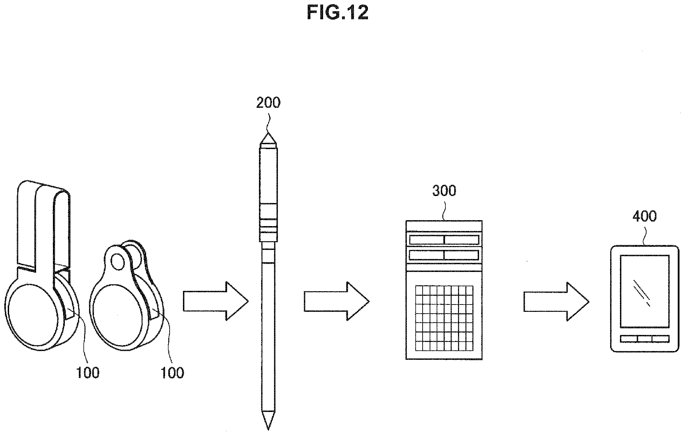

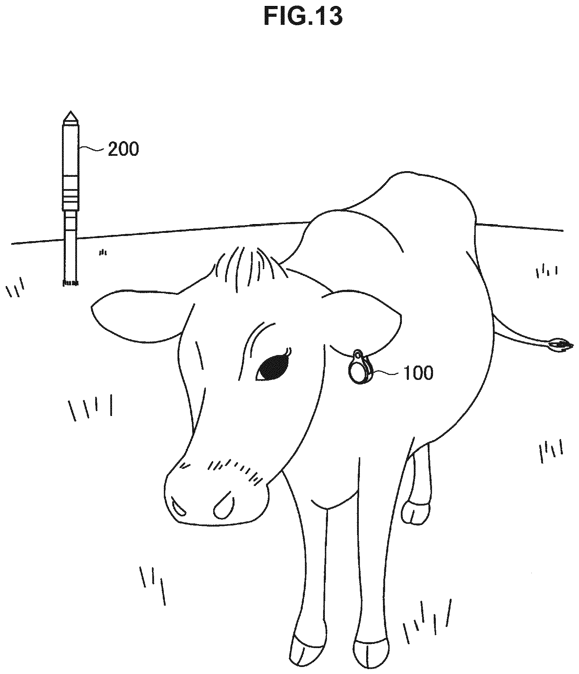

Furthermore, a specific configuration of the information processing system according to the embodiment when the attachment targets are farm animals will be described with reference to FIGS. 12 and 13. FIG 12 is an explanatory diagram illustrating a specific configuration of the information processing system when the attachment targets are farm animals, and FIG. 13 is an explanatory diagram illustrating an attachment example of the monitoring devices 100 when the attachment targets are farm animals.

As illustrated in FIGS. 12 and 13, the monitoring devices 100 are formed into a coin shape, for example, and are accommodated in covers for attachment to the farm animals when the attachment targets are farm animals. The monitoring devices 100 may be attached to a part of the bodies of the farm animals such as cattle, for example.

The receivers 200 are formed as cylindrical piles with communication modules provided at the tops thereof. The receivers 200 may be formed into a shape with a sharpened lower end so as to be able to be easily installed in the ground. The receivers 200 may be knocked into the ground of the grass land or the like at an appropriate interval so as to be able to receive signals from the monitoring devices 100 without omission, for example.

The power generation signals transmitted from the monitoring devices 100 are received by the receivers 200 first and are then transmitted from the receivers 200 to the information processing device 300. The information processing device 300 determines at least either of the states of the farm animals to which the monitoring devices 100 are attached and the environments surrounding the monitoring devices 100 on the bases of the received power generation signals. The information processing device 300 manages the information determined in relation to at least either of the states of the farm animals and the surrounding environments and generates display for referring to the managed information. In this manner, the user can unitarily manage the states of the farm animals to which the monitoring devices 100 are attached by using a device provided with a display unit, such as a mobile phone, a smart phone, a tablet terminal, or a laptop personal computer, to refer to the display generated by the information processing device 300.

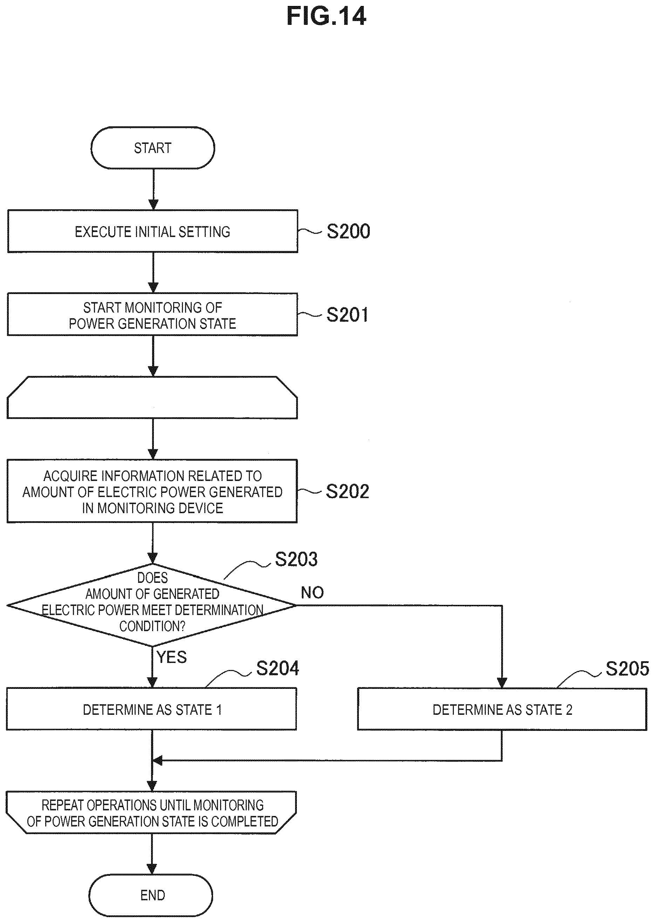

Next, a specific operation example of the aforementioned information processing system will be described with reference to FIGS. 14 to 25.

First, operations performed when the information processing device 300 determines states of the attachment targets by using the power generation signals of the monitoring devices 100 will be described with reference to FIGS. 14 to 18. FIGS. 14 to 18 are flowcharts for describing operations of determining states of the attachment targets by using the power generation signals of the monitoring devices 100.

As illustrated in FIG. 14, the information processing device 300 performs initial setting of communication protocols in the monitoring devices 100, a monitoring interval of the amounts of the generated electric power, and determination conditions for the amounts of the generated electric power first (S200). Next, the information processing device 300 starts monitoring of the power generation states of the monitoring devices 100 (S201). Here, the information processing device 300 acquires information related to the amounts of the electric power generated in the monitoring devices 100 at the set monitoring interval (S202). Subsequently, the information processing device 300 determines whether or not the amounts of the electric power generated in the monitoring devices 100 meet the determination conditions (S203). If the amounts of the generated electric power meet the determination conditions (S203/Yes), the information processing device 300 determines the state of the attachment targets as a predetermined state 1. If the amounts of the generated electric power do not meet the determination conditions (S203/No), the information processing device 300 determines the state of the attachment targets as a predetermined state 2 (S205). The information processing device 300 repeats the operations in S202 to S205 until the monitoring of the power generation states of the monitoring devices 100 is completed.

The information processing device 300 may determine the states of the attachment targets only when the amounts of variation in the amounts of the electric power generated in the monitoring devices 100 is greater than or equal to a threshold value.

Specifically, the information processing device 300 performs initial setting of communication protocols in the monitoring devices 100, a monitoring interval of the amounts of the generated electric power, a threshold value of the amounts of variation in the amounts of the generated electric power, and determination conditions for the amounts of the generated electric power first (S210). Next, the information processing device 300 starts monitoring of the power generation states of the monitoring devices 100 (S211). Here, the information processing device 300 acquires information related to the amounts of the electric power generated in the monitoring devices 100 at the set monitoring interval (S212). Subsequently, the information processing device 300 determines whether or not the amounts of variation in the amounts of the electric power generated in the monitoring devices 100 meet the determination conditions (S213). If the amounts of variation in the amounts of the generated electric power are less than the threshold value (S213/No), the information processing device 300 returns to the monitoring of the power generation states (S212) and does not determine the states of the attachment targets.

In contrast, if the amounts of variation in the amounts of the generated electric power are greater than or equal to the threshold value (S213/Yes), the information processing device 300 determines whether or not the amounts of the electric power generated in the monitoring devices 100 meet the determination conditions (S214). If the amounts of the generated electric power meet the determination conditions (S214/Yes), the information processing device 300 determines the state of the attachment targets as a predetermined state 1 (S215). In contrast, if the amounts of the generated electric power do not meet the determination conditions (S214/No), the information processing device 300 determines the state of the attachment target as a predetermined state 2 (S216). The information processing device 300 repeats the operations in S212 to S216 until the monitoring of the power generation states of the monitoring devices 100 is completed.

Furthermore, the information processing device 300 may determine the states of the attachment targets by using a plurality of determination conditions.

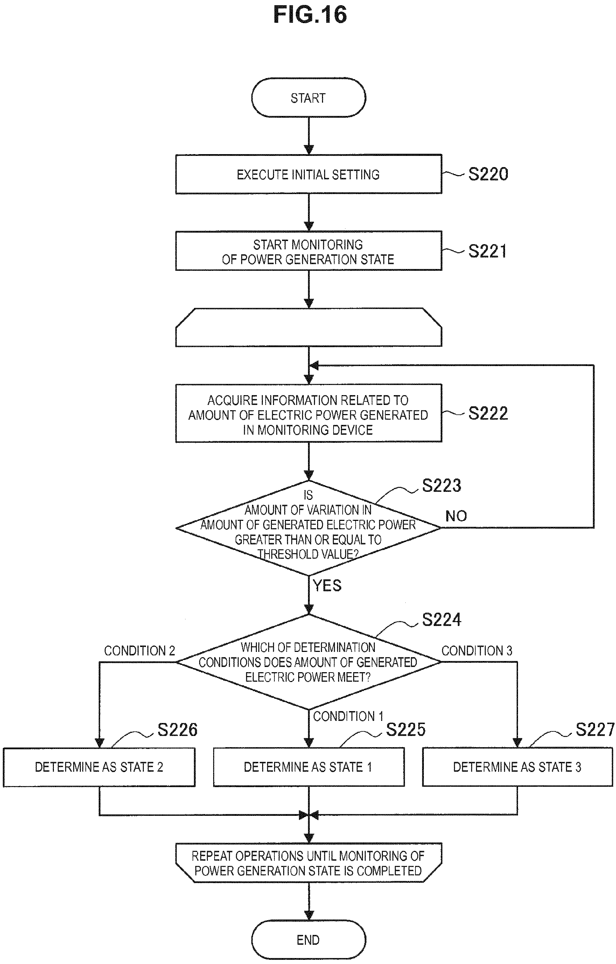

Specifically, the information processing device 300 performs initial setting of the communication protocols in the monitoring devices 100, the monitoring interval of the amounts of the generated electric power, the threshold value of the amounts of variation in the amounts of the generated electric power, and the determination conditions of the amounts of the electric power first as illustrated in FIG. 16 (S220). Next, the information processing device 300 starts the monitoring of the power generation states of the monitoring device 100 (S221). Here, the information processing device 300 acquires information related to the amounts of the electric power generated in the monitoring devices 100 at the set monitoring interval (S222). Subsequently, the information processing device 300 determines whether or not the amounts of variation in the amounts of the electric power generated in the monitoring devices 100 are greater than or equal to the threshold value (S223). If the amounts of the variation in the amounts of the generated electric power are less than the threshold value (S223/No), the information processing device 300 returns to the monitoring of the power generation states (S222) and does not determine the states of the attachment targets.

In contrast, if the amounts of variation in the amounts of the generated electric power are greater than or equal to the threshold value (S223/Yes), the information processing device 300 determines which of determination conditions 1 to 3 the amounts of the electric power generated in the monitoring devices 100 meet (S224). If the amounts of the generated electric power meet the condition 1 (S224/condition 1), the information processing device 300 determines the state of the attachment targets as a predetermined state 1 (S225). If the amounts of the generated electric power meet the condition 2 (S224/condition 2), the information processing device 300 determines the state of the attachment targets as a predetermined state 2 (S226). If the amounts of the generated electric power meet the condition 3 (S224/condition 3), the information processing device 300 determines the state of the attachment targets as a predetermined state 3 (S227). The information processing deice 300 repeats the operations in S222 to S227 until the monitoring of the power generation states of the monitoring devices 100 is completed.

The information processing device 300 may determine whether or not abnormality has occurred in the monitoring devices 100 by using an abnormality determination condition for detecting abnormality in the monitoring device 100 in addition to the state determination of the attachment targets.

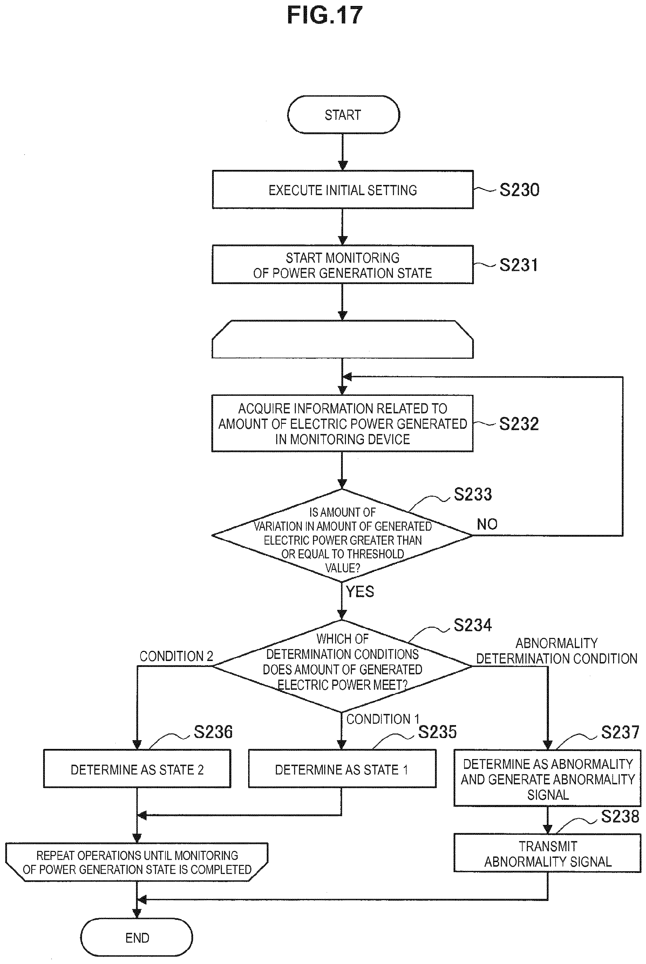

Specifically, the information processing device 300 performs initial setting of the communication protocols in the monitoring devices 100, the monitoring interval of the amounts of the generated electric power, the threshold value of the amounts of variation in the amounts of the generated electric power, and the determination conditions and an abnormality determination condition of the amounts of the generated electric power first as illustrated in FIG. 17. Next, the information processing device 300 starts the monitoring of the power generation states of the monitoring devices 100 (S231). Here, the information processing device 300 acquires information related to the amounts of the electric power generated in the monitoring devices 100 at the set monitoring interval (S232). Subsequently, the information processing device 300 determines whether or not the amounts of variation in the amounts of the electric power generated in the monitoring devices 100 are greater than or equal to the threshold value (S233). If the amounts of variation in the amounts of the generated electric power are less than the threshold value (S233/No), the information processing device 300 returns to the monitoring of the power generation states (S232) and does not determine the states of the attachment target.

In contrast, if the amounts of variation in the amounts of the generated electric power are greater than or equal to the threshold value (S233/Yes), the information processing device 300 determines which of the condition 1, the condition 2, and the abnormality determination condition the amounts of the electric power generated in the monitoring devices 100 meet (S234). If the amounts of the generated electric power meet the condition 1 (S234/condition 1), the information processing device 300 determines the state of the attachment targets as a predetermined state 1 (S235). If the amounts of the generated electric power meet the condition 2 (S234/condition 2), the information processing device 300 determines the state of the attachment targets as a predetermined state 2 (S236). If no abnormality has occurred in the monitoring devices 100, the information processing device 300 repeats the operations in S232 to S225 until the monitoring of the power generation states of the monitoring devices 100 is completed.

In contrast, if the amounts of the generated electric power meet the abnormality determination condition (S234/abnormality determination condition), the information processing device 300 determines that abnormality has occurred in the monitoring devices 100 and generates abnormality signals (S237). Subsequently, the information processing device 300 transmits the abnormality signal and notifies information that abnormality has occurred in the monitoring devices 100 to the user (S238). In such a case, the information processing device 300 completes the monitoring of the power generation states of the monitoring devices 100.

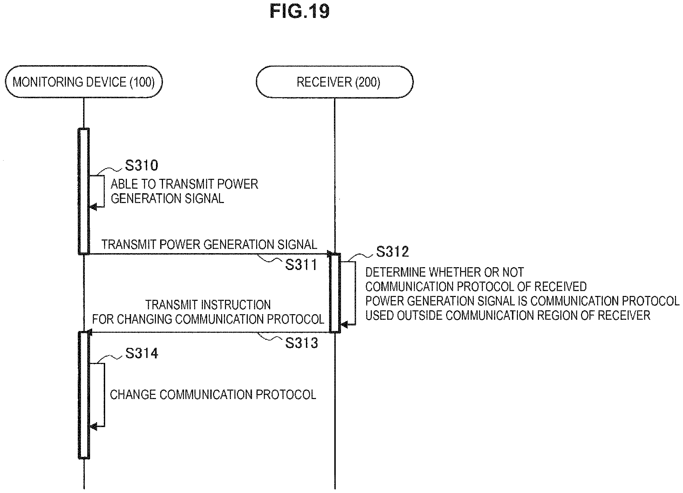

Next, operations of determining variation in an environment surrounding a monitoring device 100 on the basis of a power generation signal from the monitoring device 100 in the information processing system will be described with reference to FIGS. 19 to 21. FIGS. 19 to 21 are sequence diagrams for describing operations of determining variation in the environment surrounding the monitoring device 100 on the basis of the power generation signal from the monitoring device 100.

As illustrated in FIG. 19, a predetermined amount of generated electric power is stored in the monitoring device 100 first, and it becomes possible to transmit a power generation signal (S300). In this manner, the monitoring device 100 transmits the power generation signal to a receiver 200 (S301), and the receiver 200, which has received the power generation signal, determines whether or not the environment surrounding the monitoring device 100 has varied on the basis of the received power generation signal (S302). If it is determined that the environment surrounding the monitoring device 100 has varied, the receiver 200 transmits information related to the variation in the surrounding environment to the monitoring device 100 (S303), and the monitoring device 100, which has received the information, changes the information related to the surrounding environment, which has been stored therein (S304).

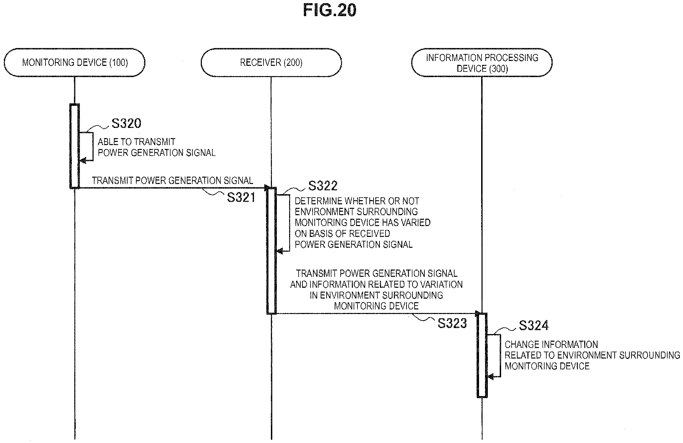

Next, the operations of determining variation in the environment surrounding the monitoring device 100 described above with reference to FIG. 19 will be more specifically described with reference to FIG. 20.

As illustrated in FIG. 20, the predetermined amount of generated electric power is stored in the monitoring device 100 first, and it becomes possible to transmit the power generation signal (S310). In this manner, the monitoring device 100 transmits the power generation signal to the receiver 200 (S311), and the receiver 200, which has received the power generation signal, determines whether or not a communication protocol of the received power generation signal is a communication protocol that is used outside a communication region of the receiver 200 (S312). If it is determined that the communication protocol of the power generation signal is a communication protocol used outside the communication region of the receiver 200, the receiver 200 transmits an instruction for changing the communication protocol to the monitoring device 100 (S313), and the monitoring device 100, which has received the information, changes the communication protocol (S314).

In such a case, the receiver 200 can control all communication protocols of transmitted power generation signals such that they are the same communication protocols and can thus uniformize the prerequisite of the power generation signals.

The receiver 200 may identify the location of the monitoring device 100 or the target to which the monitoring device 100 is attached, from the communication protocol. The receiver 200 may use the communication protocol of the transmitted power generation signal as information for identifying the environment surrounding the monitoring device 100.

The information related to variation in the environment surrounding the monitoring device 100 may be transmitted to the information processing device 300 rather than the monitoring device 100.

Specifically, a predetermined amount of generated electric power is stored in the monitoring device 100 first, and it becomes possible to transmit the power generation signal (S320) as illustrated in FIG. 20. In this manner, the monitoring device 100 transmits the power generation signal to the receiver 200 (S321). The receiver 200, which has received the power generation signal, determines whether or not the environment surrounding the monitoring device 100 has varied on the basis of the received power generation signal (S322).

If it is determined that the environment surrounding the monitoring device 100 has varied, the receiver 200 transmits the information related to the variation in the environment surrounding the monitoring device 100 and the power generation signal to the information processing deice 300 (S323). If it is determined that the environment surrounding the monitoring device 100 has not varied, the receiver 200 transmits only the power generation signal to the information processing device 300.

The information processing device 300 changes the information related to the environment surrounding the monitoring device 100, which has transmitted the power generation signal, on the basis of the received information related to the variation in the environment surrounding the monitoring device 100 (S324).

In such a case, the information processing system can find that the environment surrounding the monitoring device 100 has varied by the information processing device 300 without changing the information stored in the monitoring device 100.

Furthermore, the information related to the variation in the environment surrounding the monitoring device 100 may be transmitted to both the monitoring device 100 and the information processing device 300.

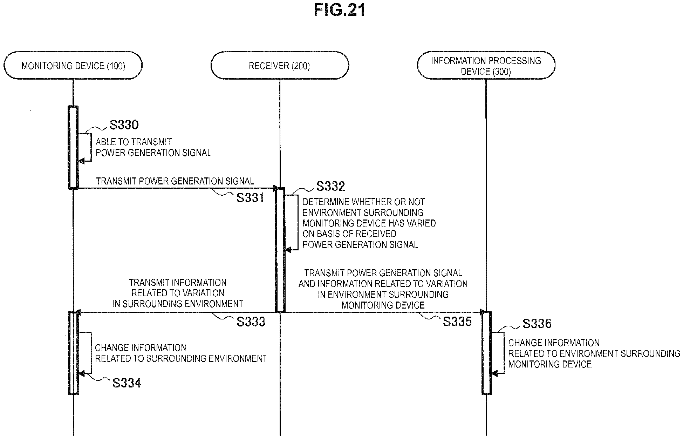

Specifically, a predetermined amount of generated electric power is stored in the monitoring device 100 first, and it becomes possible to transmit the power generation signal as illustrated in FIG 21 (S330). In this manner, the monitoring device 100 transmits the power generation signal to the receiver 200 (S331), and the receiver 200, which has received the power generation signal, determines whether or not the environment surrounding the monitoring device 100 has varied on the basis of the received power generation signal (S332).

If it is determined that the environment surrounding the monitoring device 100 has varied, the receiver 200 transmits the information related to the variation in the surrounding environment to the monitoring device 100 (S333) and transmits the information related to the variation in the environment surrounding the monitoring device 100 and the power generation signal to the information processing device 300 (S335). In this manner, the monitoring device 100, which has received the information, changes the information related to the surrounding environment, which has been stored therein (S334), and the information processing device 300, which has received the information, changes the information related to the environment surrounding the monitoring device 100 which has transmitted the power generation signal (S336).

In this case, it is possible to store the information related to the variation in the environment surrounding the monitoring device 100 in both the monitoring device 100 and the information processing device 300 and to thereby enhance conformity of the stored information.

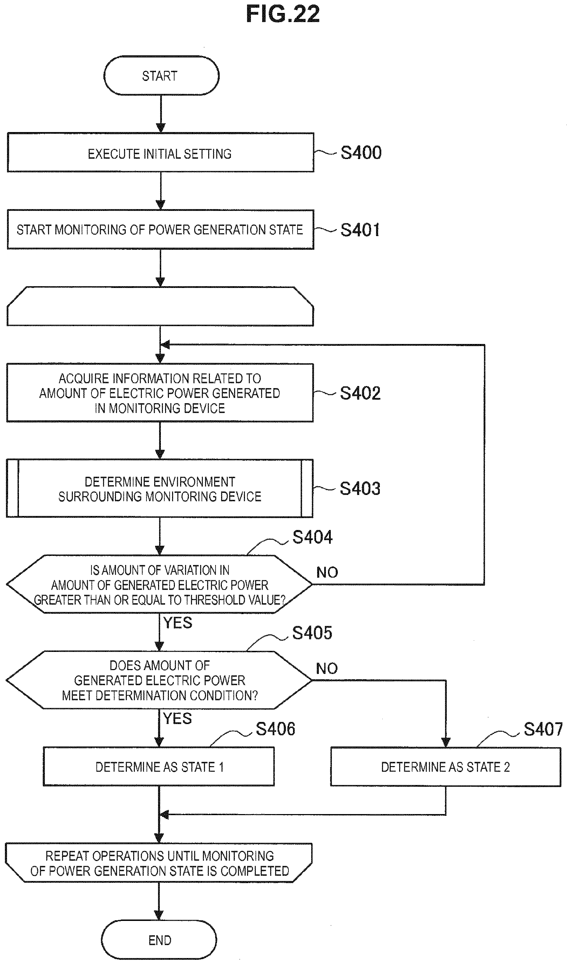

Next, operations of determining a state of an attachment target by using a power generation signal of the monitoring device 100 and information related to variation in an environment surrounding the receive 200 will be described with reference to FIGS. 22 to 24. FIGS. 22 to 24 are flowcharts for describing the operations of determining the state of the attachment target by using the power generation signal of the monitoring device 100 and the information related to the variation in the environment surrounding the receiver 200.

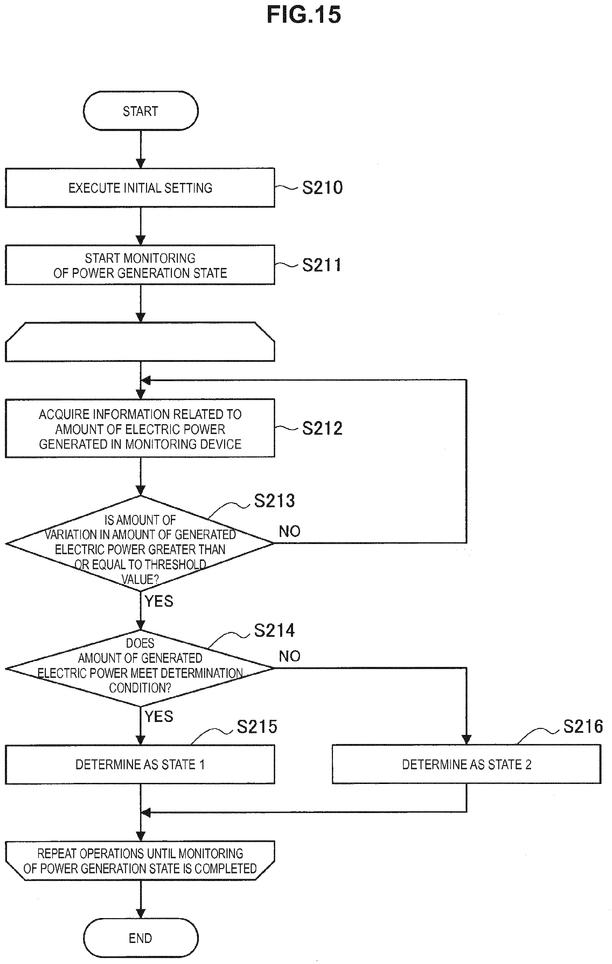

The flowchart illustrated in FIG. 22 illustrates an operation example in which an operation of determining an environment surrounding the monitoring device (S403) is inserted between S212 and S213 in the flowchart illustrated in FIG. 15, for example. The operation of determining the environment surrounding the monitoring device 100 (S403) corresponds to the operations in S322 to S324 in FIG. 20, for example.

As illustrated in FIG. 22, the information processing device 300 performs initial setting of the communication protocol in the monitoring device 100, the monitoring interval of the amount of the generated electric power, the threshold value of the amount of variation in the amount of the generated electric power, and a determination condition for the amount of the generated electric power first (S400). Next, the information processing device 300 starts the monitoring of the power generation state of the monitoring device 100. Here, the information processing device 300 acquires information related to the amount of the electric power generated in the monitoring device 100 at the set monitoring interval (S402). Here, the information processing device 300 acquires information related to variation in the environment surrounding the monitoring device 100 and determines a state of the environment surrounding the monitoring device 100 (S403).

Subsequently, the information processing device 300 determines whether or not the amount of the variation in the amount of the electric power generated in the monitoring device 100 is greater than or equal to the threshold value (S404). If the amount of the variation in the amount of the generated electric power is less than the threshold value (S404/No), the information processing device 300 returns to the monitoring of the power generation state (S402) and does not determine the state of the attachment target.

In contrast, if the amount of the variation in the amount of the generated electric power is greater than or equal to the threshold value (S404/Yes), the information processing device 300 determines whether or not the amount of the electric power generated in the monitoring device 100 meets a determination condition (S405). If the amount of the generated electric power meets the determination condition (S405/Yes), the information processing device 300 further determines the state of the attachment target as a predetermined state 1 on the basis of the state of the environment surrounding the monitoring device 100 (S406). In contrast, if the amount of the generated electric power does not meet the determination condition (S405/No), the information processing device 300 further determines the state of the attachment target as a predetermined state 2 on the basis of the state of the environment surrounding the monitoring device 100 (S406). The information processing device 300 repeats the operations in S402 to S407 until the monitoring of the power generation state of the monitoring device 100 is completed.

The information processing device 300 may determine the state of the attachment target only when the amount of the electric power generated in the monitoring device 100 is greater than or equal to the threshold value.

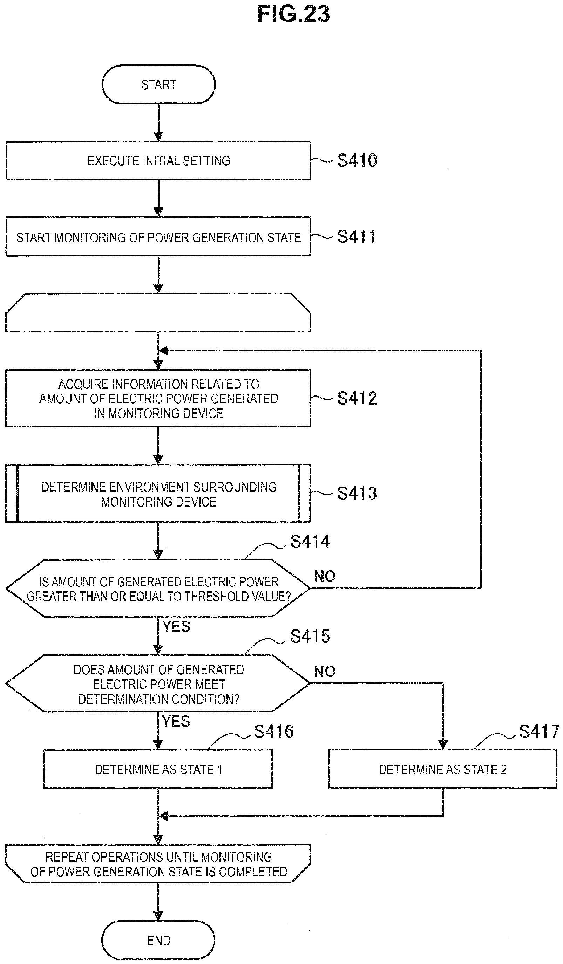

Specifically, the information processing device 300 performs initial setting of the communication protocol in the monitoring device 100, the monitoring interval of the amount of the generated electric power, the threshold of the amount of the generated electric power, and the determination condition of the amount of the generated electric power first as illustrated in FIG. 23 (S410). Next, the information processing device 300 starts the monitoring of the power generation state of the monitoring device 100 (S411). Here, the information processing device 300 acquires information related to the amount of the electric power generated in the monitoring device 100 at the set monitoring interval (S412). Here, the information processing device 300 acquires information related to variation in the environment surrounding the monitoring device 100 and determines a state of the environment surrounding the monitoring device 100 (S413).

Subsequently, the information processing device 300 determines whether or not the amount of the electric power generated in the monitoring device 100 is greater than or equal to the threshold value (S414). If the amount of the generated electric power is less than the threshold value (S414/No), the information processing device 300 returns to the monitoring of the power generation state (S412) and does not determine the state of the attachment target.

In contrast, if the amount of the generated electric power is greater than or equal to the threshold value (S414/Yes), the information processing device 300 determines whether or not the amount of the electric power generated in the monitoring device 100 meets the determination condition (S415). If the amount of the generated electric power meets the determination condition (S415/Yes), the information processing device 300 further determines the state of the attachment target as a predetermined state 1 on the basis of the state of the environment surrounding the monitoring device 100, for example (S416). In contrast, if the amount of the generated electric power does not meet the determination condition (S415/No), the information processing device 300 further determines the state of the attachment target as a predetermined state 2 on the basis of the state of the environment surrounding the monitoring device 100 (S416). The information processing device 300 repeats the operations in S412 to S417 until the monitoring of the power generation state of the monitoring device 100 is completed.

If the operation of determining the environment surrounding the monitoring device 100 is performed before determining whether or not the amount of the electric power generated in the monitoring device 100 meets the determination condition as described above, it is possible to increase the number of times the operation of determining the environment surrounding the monitoring device 100 is performed. Therefore, the information processing device 300 can more precisely determine the environment surrounding the monitoring device 100.

The information processing device 300 may estimate that the environment surrounding the monitoring device 100 has varied when the amount of the electric power generated in the monitoring device 100 meets the determination condition, and perform the operation of determining the environment surrounding the monitoring device 100. The operation of determining the environment surrounding the monitoring device 100 (S425) corresponds to the operations in S322 to S324 in FIG. 20, for example.

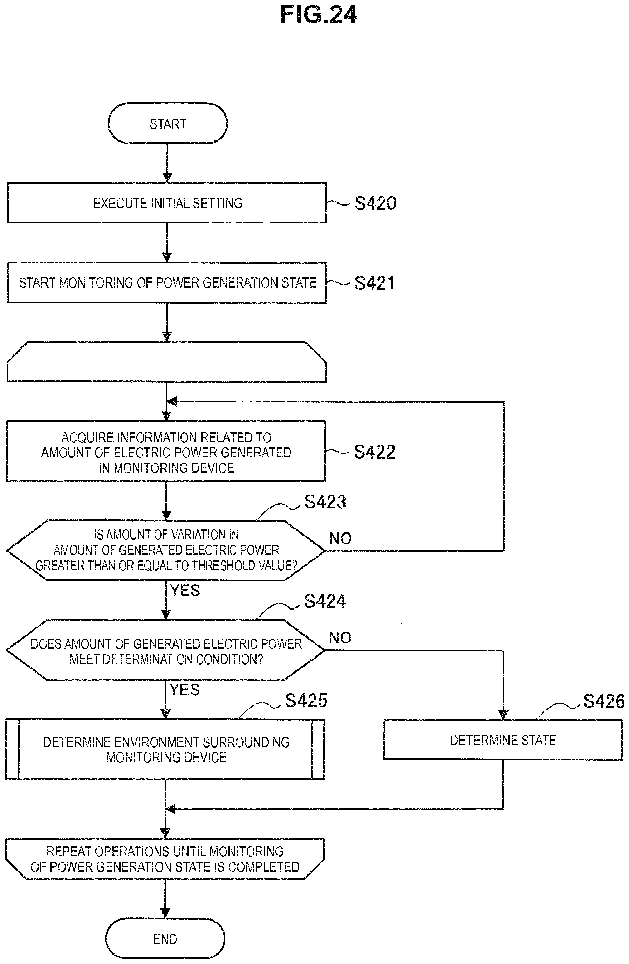

Specifically, the information processing device 300 performs initial setting of the communication protocol in the monitoring device 100, the monitoring interval of the amount of the generated electric power, the threshold value of the amount of variation in the amount of the generated electric power, and the determination condition of the amount of the generated electric power first as illustrated in FIG. 24 (S420). Next, the information processing device 300 starts the monitoring of the power generation state of the monitoring device 100 (S421). Here, the information processing device 300 acquires information related to the amount of the electric power generated in the monitoring device 100 at the set monitoring interval (S422).

Subsequently, the information processing device 300 determines whether or not the amount of the variation in the amount of the electric power generated in the monitoring device 100 is greater than or equal to the threshold value (S422). If the amount of the variation in the amount of the generated electric power is less than the threshold value (S422/No), the information processing device 300 returns to the monitoring of the power generation state (S422) and does not determine the state of the attachment target.

In contrast, if the amount of the variation in the amount of generated electric power is greater than or equal to the threshold value (S423/Yes), the information processing device 300 determines whether or not the amount of the electric power generated in the monitoring device 100 meets the predetermined condition (S424). If the amount of the generated electric power meets the predetermined condition (S424/Yes), the information processing device 300 estimates that the environment surrounding the monitoring device 100 has varied, and determines the state of the environment surrounding the monitoring device 100 (S425).

If the amount of the generated electric power does not meet the predetermined condition (S424/No), the information processing device 300 estimates that the environment surrounding the monitoring device 100 has not varied, and determines the state of the attachment target on the basis of the amount of the electric power generated in the monitoring device 100 (S426). The information processing device 300 repeats the operations in S422 to S426 until the monitoring of the power generation state of the monitoring device 100 is completed.

The information processing device 300 may determine the state of the attachment target only when the amount of the electric power generated in the monitoring device 100 is greater than or equal to the threshold value.

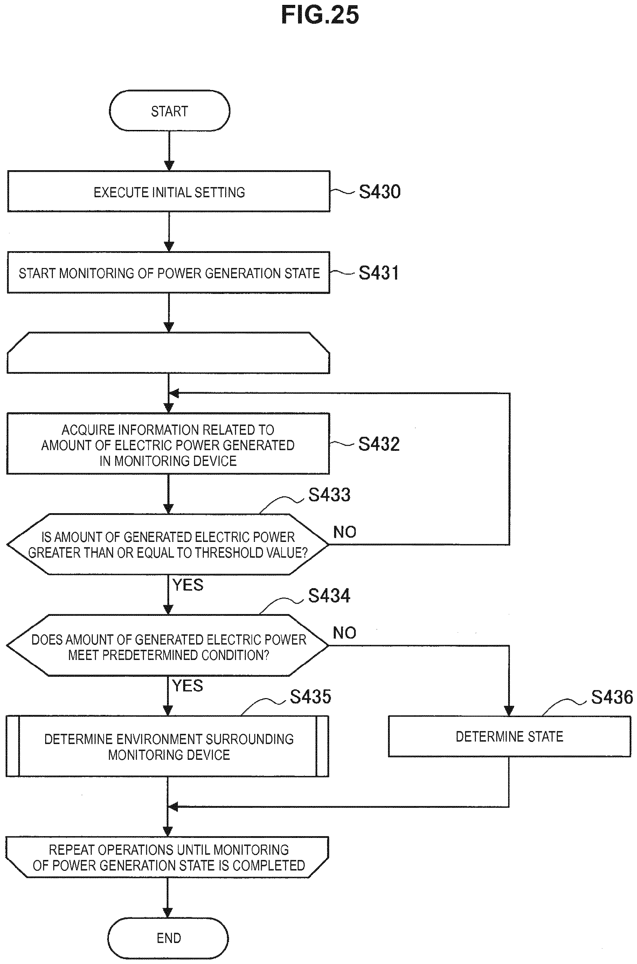

Specifically, the information processing device 300 performs initial setting of the communication protocol in the monitoring device 100, the monitoring interval of the amount of the generated electric power, the threshold value of the amount of the generated electric power, and the determination condition of the amount of the generated electric power first as illustrated in FIG. 25 (S430). Next, the information processing device 300 starts the monitoring of the power generation state of the monitoring device 100 (S431). Here, the information processing device 300 acquires information related to the amount of the electric power generated in the monitoring device 100 at the set monitoring interval (S432).

Subsequently, the information processing device 300 determines whether or not the amount of the electric power generated in the monitoring device 100 is greater than or equal to the threshold value (S433). If the amount of the generated electric power is less than the threshold value (S433/No), the information processing device 300 returns to the monitoring of the power generation state (S432) and does not determine the state of the attachment target.

In contrast, if the amount of the generated electric power is greater than or equal to the threshold value (S433/Yes), the information processing device 300 determines whether or not the amount of the electric power generated in the monitoring device 100 meets the predetermined condition (S434). If the amount of the generated electric power meets the predetermined condition (S434/Yes), the information processing device 300 estimates that the environment surrounding the monitoring device 100 has varied, and determines the state of the environment surrounding the monitoring device 100 (S435).

If the amount of the generated electric power does not meet the predetermined condition (S434/No), the information processing device 300 estimates that the environment surrounding the monitoring device 100 has not varied, and determines the state of the attachment target on the basis of the amount of the electric power generated in the monitoring device 100 (S436). The information processing device 300 repeats the operations in S432 to S436 until the monitoring of the power generation state of the monitoring device 100 is completed.

If it is determined whether or not the amount of the electric power generated in the monitoring device 100 meets the predetermined condition and the operation of determining the environment surrounding the monitoring device 100 is then performed as described above, it is possible to reduce the number of times the operation of determining the environment surrounding the monitoring device 100 is performed. Therefore, it is possible to reduce the amount of information processing and electric power consumption in the information processing device 300.

### <<3. Hardware configuration example of information processing device>>

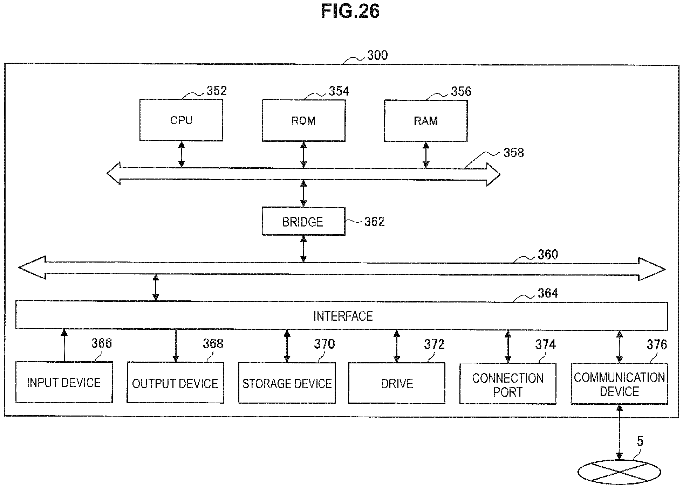

Hereinafter, an exemplary hardware configuration of the information processing device included in the information processing system according to the embodiment will be described with reference to FIG. 26. FIG 26 is a block diagram illustrating a hardware configuration example of the information processing device 300 according to the embodiment. Information processing performed by the information processing device 300 according to the embodiment is realized by cooperation between software and hardware.

As illustrated in FIG. 26, the information processing device 300 includes a CPU 352, a ROM 354, a RAM 356, a bridge 362, internal buses 358 and 360, an interface 364, an input device 366, an output device 368, a storage device 370, a drive 372, a connection port 374, and a communication device 376.

The CPU 352 functions as a computation processing device and a control device and generally controls the operations of the information processing device 300 in accordance with various programs stored in the ROM 354 or the like. The ROM 354 stores computation parameters and programs to be used by the CPU 352, and the RAM 356 temporarily stores programs to be used in execution by the CPU 352 and parameters and the like that appropriately vary during the execution. For example, the CPU 352 may execute functions of the determination unit 304 or the like.

The CPU 352, the ROM 354, and the RAM 356 are connected to each other by the bridge 362, the internal buses 358 and 360, and the like. The CPU 352, the ROM 354, and the RAM 356 are also connected to the input device 366, the output device 368, the storage device 370, the drive 372, the connection port 374, and the communication device 376 via the interface 364.

The input device 366 includes input devices, such as a touch panel, a keyboard, a mouse, a button, a microphone, a switch, and a lever, to which various kinds of information is input. The input device 366 also includes an input control circuit or the like that generates an input signal on the basis of the input information and outputs the input signal to the CPU 352.

The output device 368 includes, for example, display devices such as a cathode ray tube (CRT) display device, a liquid crystal display device, and an organic electroluminessennce (EL) display device and sound output devices such as a speaker and headphones.

The storage device 370 is a device for storing data, which is configured as an example of a storage device in the information processing device 300. The storage device 370 may include a storage medium, a storage device that stores data in the storage medium, a reading device that reads the data from the storage medium, and a deletion device that deletes the stored data.

The drive 372 is a read-writer for a storage medium and is built into or externally attached to the information processing device 300. For example, the drive 372 reads information stored in a removable storage medium attached thereto, such as a magnetic disk, an optical disc, a magneto-optical disc, or a semiconductor memory and outputs the information to the RAM 356. The drive 372 can also write information in the removable storage medium.

The connection port 374 is a connection interface configured of a connection port to which an external connection device such as a USB port, an Ethernet port, an IEEE80671 standard port, or an optical audio terminal, for example is connected. For example, the connection port 374 may execute the functions of the output unit 306.

The communication device 376 is a communication interface that is configured of a communication device for establishing connection with the communication network 5, for example. The communication device 376 may be a cable communication device that performs wired cable communication or may be a communication device that is compatible with a wired or wireless LAN. For example, the communication device 376 may execute the functions of the power generation signal acquisition unit 302.

It is also possible to create a computer program for causing hardware such as the CPU, the ROM, and the RAM built into the information processing device 300 to perform functions equivalent to those of the aforementioned respective configurations in the information processing device 300. A storage medium that stores the computer program is also provided.

### <<4. Conclusion>>

As described above, the monitoring device 100 according to the embodiment of the present disclosure includes the power generation device that varies the amount of generated electric power depending on a state of an attachment target or a surrounding environment. In this manner, the information processing device and the information processing system according to the embodiment can determine at least either of the states of the attachment target to which the monitoring device 100 is attached and the surrounding environment without using various sensors that consume electric power.

In this manner, the information processing device and the information processing system according to the embodiment can monitor the states of the attachment target, the surrounding environment, and the like without considering time restriction and depletion of stored electric power.

The preferred embodiment(s) of the present disclosure has/have been described above with reference to the accompanying drawings, whilst the present disclosure is not limited to the above examples. A person skilled in the art may find various alterations and modifications within the scope of the appended claims, and it should be understood that they will naturally come under the technical scope of the present disclosure.

Although the above embodiments were basically described on the basis of the assumption that the attachment target was a human, for example, the present technology is not limited to such an example. For example, the attachment target may be an animal such as a pet or a farm animal.

Further, the effects described in this specification are merely illustrative or exemplified effects, and are not limitative. That is, with or in the place of the above effects, the technology according to the present disclosure may achieve other effects that are clear to those skilled in the art from the description of this specification.

Additionally, the present technology may also be configured as below.
(1) An information processing device including:
   a power generation signal acquisition unit configured to acquire a power generation signal based on power generation by at least one power generation device; and
   a determination unit configured to determine at least either of states of an environment surrounding the power generation device and an attachment target to which the power generation device is attached, on the basis of the power generation signal.
(2) The information processing device according to (1),
   wherein the power generation signal includes information related to whether electric power generated by the power generation device has exceeded a threshold value.
(3) The information processing device according to (2),
   wherein the power generation signal is generated when the electric power generated by the power generation device has exceeded the threshold value, and
   the determination unit determines at least either of the states of the environment surrounding the power generation device and the attachment target to which the power generation device is attached, on the basis of a generation interval of the power generation signal.
(4) The information processing device according to any one of (1) to (3), further including:
   an output unit configured to output information related to response to the attachment target on the basis of the determination by the determination unit.
(5) An information processing system including:
   a monitoring device that includes at least one power generation device; and
   an information processing device that includes a power generation signal acquisition unit configured to acquire a power generation signal based on power generation by the power generation device, and a determination unit configured to determine at least either of states of an environment surrounding the monitoring device and an attachment target to which the monitoring device is attached, on the basis of the power generation signal.
(6) The information processing system according to (5),
   wherein the monitoring device further includes a storage device configured to individually store power generation signals based on the power generation.
(7) The information processing system according to (6),
   wherein the storage device has a storage region shared by the respective at least one power generation device by time-division or interruption processing.
(8) The information processing system according to (5),
   wherein the monitoring device includes a plurality of power generation devices, and
   the monitoring device further includes a storage device that stores power generation signals based on the power generation for the respective plurality of power generation devices.
(9) The information processing system according to (8),
   wherein the storage device has a storage region shared by the plurality of power generation devices by time-division or interruption processing.
(10) The information processing system according to any one of (5) to (9),
   wherein the power generation device supplies generated electric power at least to the monitoring device.
(11) The information processing system according to any one of (5) to (10),
   wherein the information processing system includes a switch circuit that is driven on the basis of electric power generated by the power generation device, a storage device that stores a power generation signal generated by the switch circuit, an external output device that outputs the power generation signal stored on the storage device, and a computation device that performs signal processing on the power generation signal.
(12) The information processing system according to any one of (5) to (11),
   wherein the power generation device is one of or a combination of two or more among a vibration power generation device, a kinetic power generation device, a solar power generation device, a thermoelectric conversion power generation device, an enzymatic power generation device, a radio wave power generation device, a power generation device that includes a near electromagnetic field power generation device, and a power transmission device using magnetic resonance or electromagnetic induction.
(13) The information processing system according to any one of (5) to (12),
   wherein the monitoring device further includes an electrical storage device that stores electric power generated by the power generation device.
(14) The information processing system according to (13),
   wherein the monitoring device further includes a management unit that manages the electric power stored in the electrical storage device, and
   the management unit determines whether or not electric power that exceeds a threshold value for generating the power generation signal has been stored in the electrical storage device.
(15) The information processing system according to any one of (5) to (14),
   wherein the monitoring device further includes a control unit that controls an input from an outside to the monitoring device and an output from the monitoring device to the outside.
(16) An information processing method including:
   acquiring a power generation signal based on power generation by at least one power generation device; and
   determining, by a computation processing device, at least either of states of an environment surrounding the power generation device and an attachment target to which the power generation device is attached, on the basis of the power generation signal.
(17) A program that causes a computer to function as:
   a power generation signal acquisition unit configured to acquire a power generation signal based on power generation by at least one power generation device; and
   a determination unit configured to determine at least either of states of an environment surrounding the power generation device and an attachment target to which the power generation device is attached, on the basis of the power generation signal.

## Claims

1. An information processing device comprising:
a power generation signal acquisition unit configured to acquire a power generation signal based on power generation by at least one power generation device; and
a determination unit configured to determine at least either of states of an environment surrounding the power generation device and an attachment target to which the power generation device is attached, on the basis of the power generation signal.

2. The information processing device according to claim 1,
wherein the power generation signal includes information related to whether electric power generated by the power generation device has exceeded a threshold value.

3. The information processing device according to claim 2,
wherein the power generation signal is generated when the electric power generated by the power generation device has exceeded the threshold value, and
the determination unit determines at least either of the states of the environment surrounding the power generation device and the attachment target to which the power generation device is attached, on the basis of a generation interval of the power generation signal.

4. The information processing device according to claim 1, further comprising:
an output unit configured to output information related to response to the attachment target on the basis of the determination by the determination unit.

5. An information processing system comprising:
a monitoring device that includes at least one power generation device; and
an information processing device that includes a power generation signal acquisition unit configured to acquire a power generation signal based on power generation by the power generation device, and a determination unit configured to determine at least either of states of an environment surrounding the monitoring device and an attachment target to which the monitoring device is attached, on the basis of the power generation signal.

6. The information processing system according to claim 5,
wherein the monitoring device further includes a storage device configured to individually store power generation signals based on the power generation.

7. The information processing system according to claim 6,
wherein the storage device has a storage region shared by the respective at least one power generation device by time-division or interruption processing.

8. The information processing system according to claim 5,
wherein the monitoring device includes a plurality of power generation devices, and
the monitoring device further includes a storage device that stores power generation signals based on the power generation for the respective plurality of power generation devices.

9. The information processing system according to claim 8,
wherein the storage device has a storage region shared by the plurality of power generation devices by time-division or interruption processing.

10. The information processing system according to claim 5,
wherein the power generation device supplies generated electric power at least to the monitoring device.

11. The information processing system according to claim 5,
wherein the information processing system includes a switch circuit that is driven on the basis of electric power generated by the power generation device, a storage device that stores a power generation signal generated by the switch circuit, an external output device that outputs the power generation signal stored on the storage device, and a computation device that performs signal processing on the power generation signal.

12. The information processing system according to claim 5,
wherein the power generation device is one of or a combination of two or more among a vibration power generation device, a kinetic power generation device, a solar power generation device, a thermoelectric conversion power generation device, an enzymatic power generation device, a radio wave power generation device, a power generation device that includes a near electromagnetic field power generation device, and a power transmission device using magnetic resonance or electromagnetic induction.

13. The information processing system according to claim 5,
wherein the monitoring device further includes an electrical storage device that stores electric power generated by the power generation device.

14. The information processing system according to claim 13,
wherein the monitoring device further includes a management unit that manages the electric power stored in the electrical storage device, and
the management unit determines whether or not electric power that exceeds a threshold value for generating the power generation signal has been stored in the electrical storage device.

15. The information processing system according to claim 5,
wherein the monitoring device further includes a control unit that controls an input from an outside to the monitoring device and an output from the monitoring device to the outside.

16. An information processing method comprising:
acquiring a power generation signal based on power generation by at least one power generation device; and
determining, by a computation processing device, at least either of states of an environment surrounding the power generation device and an attachment target to which the power generation device is attached, on the basis of the power generation signal.

17. A program that causes a computer to function as:
a power generation signal acquisition unit configured to acquire a power generation signal based on power generation by at least one power generation device; and
a determination unit configured to determine at least either of states of an environment surrounding the power generation device and an attachment target to which the power generation device is attached, on the basis of the power generation signal.
